# EUROPEAN PATENT SPECIFICATION

(11) **EP 1 806 377 B1**
(45) Date of publication and mention of the grant of the patent: **05.09.2012**
(21) Application number: 05795884.5
(22) Date of filing: 20.10.2005
(51) Int. Cl.: C08G 63/06, C07K 14/00, C08G 69/44, A61K 47/42, C07K 11/00

(54) **TEMPERATURE-RESPONSIVE DEPSIPEPTIDE POLYMER**
TEMPERATUREMPFINDLICHES DEPSIPEPTIDPOLYMER
POLYMÈRE THERMOSENSIBLE DE TYPE DEPSIPEPTIDE

(30) Priority: 20.10.2004 JP 2004305953
(43) Date of publication of application: 11.07.2007
(73) Proprietor: National University Corporation Gunma University, Gunma 371-0044 (JP)
(72) Inventor: OKU, Hiroyuki, Maebashi-shi, Gunma 3710007 (JP); SHICHIRI, Kazuaki, a 5290354; (JP); TAIRA, Tomohiro, Yazaki Industrial Chemical Co., Shizuoka-shi, Shizuoka 4228021 (JP); INOUE, Aya, Asuka Haitsu II, Kiryu-shi, Gunma 3760001 (JP); YAMADA, Keiichi, Kiryu-shi, Gunma 3760035 (JP); KATAKAI, Ryoichi, 3760001 (JP)
(74) Representative: Gillet, Raphaëlle
(86) International application number: PCT/JP2005/019332
(87) International publication number: WO 2006/043644

(56) References cited:
- JP-A- 5 112 468
- JP-A- 7 278 277
- JP-A- 8 311 174
- JP-A- 10 007 583
- JP-A- 2001 187 749
- TAIRA ET AL: "Synthesis and properties of an elastin model depsipeptide sequences, containing-Gly-Val-Gly-Hmb-Pro- (Hmb = 2-hydroxy-3-methylbutanoic acid)" PEPTIDE SCIENCE, PROTEIN RESEARCH FOUNDATION, MINOO,, JP, 2003, pages 177-180, XP009097247 ISSN: 1344-7661
- SHICHIRI ET AL: "Synthesis and properties of a thermo-responsible polydepsipeptide containingHmb (2-hydroxy-3-methylbutanoic acid) residues" PEPTIDE SCIENCE, PROTEIN RESEARCH FOUNDATION, MINOO,, JP, 2005, pages 633-636, XP009097248 ISSN: 1344-7661
- DATABASE WPI Week 200131 Derwent Publications Ltd., London, GB; AN 2001-294259 XP002472996 & JP 2001 031762 A (SHARP KK) 6 February 2001 (2001-02-06)

### SEQUENCE LISTING

<110> National University Corporation Gunma University
<120> TEMPERATURE RESPONSIVE DEPSIPEPTIDE POLYMER
<130> IPF16020-C5209
<150> JP2004-305953
   <151> 2004-10-20
<160> 9
<170> Patent In version 3. 1
<210> 1
   <211> 5
   <212> PRT
   <213> Artificial sequence
<220>
   <223> artificial peptide
<220>
   <221> MISC_FEATURE
   <222> (2)
   <223> Xaa=any kind of α - amino acid
<220>
   <221> MISC_FEATURE
   <222> (4)
   <223> Xaa=valine or isoleucine
<400> 1
   Gly Xaa Gly Xaa Pro
   1 5
<210> 2
   <211> 6
   <212> PRT
   <213> Artificial sequence
<220>
   <223> artificial peptide
<400> 2
   Gly Val Gly Val Ala Pro
   1 5
<210> 3
   <211> 5
   <212> PRT
   <213> Artificial sequence
<220>
   <223> artificial peptide
<220>
   <221> MISC_FEATURE
   <222> (1)
   <223> Xaa= N- a -t-butoxycarbonyl-N-ε-benzyloxycarbonyl-L-lysine
<220>
   <221> MISC_FEATURE
   <222> (5)
   <223> Xaa= L-proline α-methyl ester
<400> 3
   Xaa Gly Val Ala Xaa
   1 5
<210> 4
   <211> 4
   <212> PRT
   <213> Artificial sequence
<220>
   <223> artificial peptide
<220>
   <221> MISC_FEATURE
   <222> (4)
   <223> Xaa= L-proline α-methyl ester
<400> 4
   Gly Val Ala Xaa
   1
<210> 5
   <211> 6
   <212> PRT
   <213> Artificial sequence
<220>
   <223> artificial peptide
<220>
   <221> MISC_FEATURE
   <222> (1)
   <223> Xaa= N-α-t-butoxycarbonyl-glycine
<220>
   <221> MISC_FEATURE
   <222> (2)
   <223> Xaa= N-ε-benzyloxycarbonyl-L-lysine
<220>
   <221> MISC_FEATURE
   <222> (6)
   <223> Xaa= L-proline α-methyl ester
<400> 5
   Xaa Xaa Gly Val Ala Xaa
   1 5
<210> 6
   <211> 5
   <212> PRT
   <213> Artificial sequence
<220>
   <223> artificial peptide
<220>
   <221> MISC_EATURE
   <222> (1)
   <223> Xaa= N-ε-benzyloxycarbonyl-L-lysine
<220>
   <221> MISC_FEATURE
   <222> (5)
   <223> Xaa= L-proline α-methyl ester
<400> 6
   Xaa Gly Val Ala Xaa
   1 5
<210> 7
   <211> 6
   <212> PRT
   <213> Artificial sequence
<220>
   <223> artificial peptide
<220>
   <221> MISC_FEATURE
   <222> (1)
   <223> Xaa= N-α-t-butoxycarbonyl-glycine
<220>
   <221> MISC_FEATURE
   <222> (2)
   <223> Xaa= N-ε-benzyloxycarbonyl-L-lysine
<400> 7
   Xaa Xaa Gly Val Ala Pro
   1 5
<210> 8
   <211> 6
   <212> PRT
   <213> Artificial sequence
<220>
   <223> artificial peptide
<220>
   <221> MISC_FEATURE
   <222> (1)
   <223> Xaa= N-α-t-butoxycarbonyl-glycine
<220>
   <221> MISC_FEATURE
   <222> (2)
   <223> Xaa= N-ε-benzyloxycarbonyl-L-lysine
<220>
   <221> MISC_FEATURE
   <222> (6)
   <223> Xaa= L-proline α-succinimide
<400> 8
   Xaa Xaa Gly Val Ala Xaa
   1 5
<210> 9
   <211> 6
   <212> PRT
   <213> Artificial sequence
<220>
   <223> artificial peptide
<220>
   <221> MISC_FEATURE
   <222> (2)
   <223> Xaa= N-ε-benzyloxycarbonyl-L-lysine
   z
<221> V1ISC FEATURE
   <222> (6)
   <223> Xaa= L-proline α-succinimide
<400> 9
   Gly Xaa Gly Val Ala Xaa
   1 5

## Description

### Technical Field

The present invention relates to a novel temperature responsive material. In particular, the present invention relates to a temperature responsive polymer composed of a depsipeptide prepared by dehydration and condensation of valic acids and amino acids. The polymer of the present invention has a property of aggregating in response to temperature of water or a buffer solution. Accordingly, the polymer of the present invention is useful for constituting bioabsorbable compositions, environment-decomposable compositions, cell adhesives, microcapsules, biological machines, biosensors, separation membranes, diagnostic kits, and the like.

### Background Art

### (1) Current state of depsipeptides and related materials

### (1a: Materials using amino acid)

A number of investigations have been conducted on polyamino acids as model substances of proteins. A polyamino acid can be easily obtained by, for example, ring opening polymerization of an *N*-carboxyamino acid anhydride that is obtained by reaction between an amino acid and phosgene (Non-patent Document 1). Since the amino acids are compounds derived from nature and are thus thought to be easily decomposed in living bodies, there have been conducted investigations on utilization of the polyamino acids as materials decomposable in living bodies and soil (Non-patent Document 2).

The polyamino acids and oligoamino acids are polymers each having a main chain linked by amide bonds as represented by the Formula 2, and are characterized by having a stable higher-order structure in which strong hydrogen bonds are formed between molecules or in molecules. Many investigations have revealed that easiness in preparation of the polyamino acids and oligoamino acids and biodecomposition property thereof are closely related to the strength of interactions between the molecules and in the molecules.

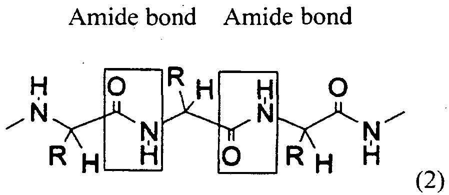

Actually, polyamino acids and oligoamino acids having non-polar side chains cannot be molded by a melting method or a solution method. Meanwhile, polyamino acids comprising polar amino acids having protected side chains, such as aspartic acid and glutamic acid can be molded by melting, and are relatively soluble in organic solvents because formation of the strong hydrogen bonds are inhibited and the polyamino acids can thus be molded (Non-patent Document 3).

However, thus-molded materials have a defect that the speed of biodecomposition is slow. For example, in a case where the material is subcutaneously implanted to a rat, only 35% of the material is decomposed after 90 days even at the soonest (Non-patent Document 4). Thus, it was found that the polyamino acids and oligoamino acids having strong interactions between molecules or in molecules due to the hydrogen bonds between amide bonds in the main chains thereof are unsuitable for the biological materials from viewpoints of molding and decomposition property.

### (1b: Materials using hydroxyl acid)

Polyhydroxy acids and oligohydroxy acids are substances each having a main chain that is linked by ester bonds as represented by the Formula 3. The ester bonds cannot form the hydrogen bonds, so the polyhydroxy acids do not have the strong interactions between molecules or in molecules. Accordingly, the polyhydroxy acids are expected to exhibit more excellent decomposition property in living bodies than the polyamino acids.

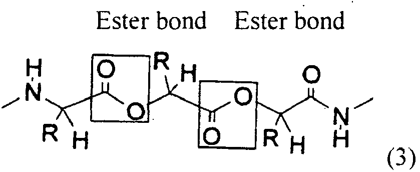

Actually, it is relatively easy to mold polyhydroxy acids having relatively low molecular weights (around 10,000) by melting. In addition, the decomposition speed in living bodies widely varies depending on the molecular weights, and the decomposition speed of the polyhydroxy acids having lower molecular weight is high. In addition, the polyhydroxy acids are completely decomposed and eliminated from the living bodies (Non-patent Document 5).

Based on the above-mentioned properties, the polyhydroxy acids such as polylactic acid and poly(lactic acid-*co*-glycolic acid) (the term "-*co*-" indicates a copolymer compound) have been practically used as biological materials for clinical purposes. Meanwhile, defects of the polyhydroxy acids have been come out. For example, the polyhydroxy acids have the following three defects: the polyhydroxy acids release a large amount of an acid component during decomposition, which results in generation of inflammation in the living bodies; only acid-resistant substances can be used as biological materials; and the polyhydroxy acids have low mechanical strength because substantially no interactions between molecules and in molecules are present.

### (1c: Materials using hydroxy acid and amino acid)

The depsipeptides are polymers or oligomers each having a main chain that is linked by ester bonds and amide bonds as represented by the Formula 4. The skeleton of its structure is composed of amide bonds and ester bonds.

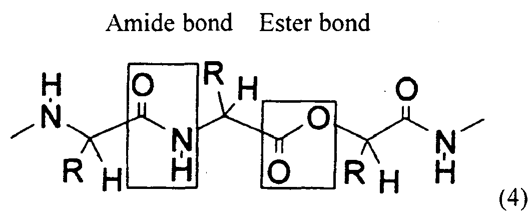

Structural stability and stiffness due to mechanical strength are expected owing to strong interactions between molecules and in molecules caused by the amide bonds in the skeleton. Meanwhile, structural instability and flexibility due to reduction in mechanical strength are expected since the ester bonds provide weak interactions or are apt to repel with one another. Accordingly, the depsipeptides can be used for materials having characteristics of the oligomer or polymer of the amino acids and hydroxy acids. In other words, materials having a wide spectrum of properties can be synthesized by changing the kinds, composition, and sequence of the amino acids and the hydroxy acids. As described above, the depsipeptides are extremely attractive substances.

Various investigations have been actually conducted on the depsipeptide. For example, it was revealed that the decomposition speed of the depsipeptide in living bodies can be controlled within a wide range of 2 weeks up to 6 months by changing hydrophobic property and level of steric hindrance by changing the side chain to H-, CH₃-, (CH₃)₂CH-, or (CH₃)₂CH-CH₂-. In addition, the depsipeptide has a characteristic that it does not cause inflammation on a contact surface of a living tissue (Non-patent Document 6).

### (2) Current status of temperature responsive materials:

In recent years, investigations on temperature responsive materials which are aggregated by increasing temperature have been attracting attention. These temperature responsive materials contain large amounts of water, and thus are expected to be utilized for wound-covering materials, microcapsules, biological machines, biosensors, separation membranes, and the like.

### (2a: vinyl polymer)

Among the temperature responsive materials, those on which most intensive investigations are conducted are materials containing as a main component a vinyl polymer such as poly(*N*-substituted methacrylamide) or poly(*N*-substituted acrylamide) (for example, Patent Documents 1 to 6). Since the vinyl polymer cannot be decomposed in the living bodies or soil, investigations have been vigorously conducted on amelioration of this property. For example, there have been used copolymers of the vinyl polymer with starch (Patent Document 7), dextran (Patent Document 8), and polyethylene glycol or polypropylene glycol (Patent Document 9), but there is still a problem in that the vinyl oligomers are not decomposed and remains in the living bodies or soil.

### (2b: other materials)

Although the wide spectrum of characteristics in the preparation property and decomposition property cannot be provided by changing the kind, composition, or sequence as in the depsipeptides, there are known some materials which can be decomposed into safe compounds in the living bodies or soil. Examples thereof include polyether ester (Patent Document 10), methyl cellulose (Patent Document 11), poly(*N*-substituted asparagine) (Patent Document 12), and starch or an alginic acid-peptide copolymer (Patent Document 13). In particular, materials made of methyl cellulose are practically used as components of commercially-available eye drops.

Methods involving gene recombination which have developed in recent years are also used for development of the temperature responsive materials (Patent Documents 14 and 15). The methods are based on synthesis of a model substance of a protein called "elastin" and investigations on the structure thereof (Non-patent Document 7). In these methods, an amino acid sequence or composition can be changed so that a temperature responsive material having a wide spectrum of properties can be provided. An elementary sequence mainly used is -Gly-Aaa-Gly-Baa-Pro- (SEQ ID NO: 1, provided that Aaa may be almost all kinds of α-amino acids including valine, and Baa is valine or isoleucine). As a problem when the temperature responsive material is used as a biological material, contamination of impurities derived from bacterial cells which are called as "pyrogen" has to be prevented when the bacterial cells are disrupted for extraction and purification. In addition, a large-scale culture appliance larger than several hundred liters is required for synthesis in few grams order.

### (3) Investigation on synthesis of temperature responsive depsipeptide and problems thereof:

The inventors of the present invention have reported an aspect of the depsipeptide unit to be used in implementation of the present invention, that is, -Gly-Val-Gly-Hmb-Pro-(Hmb = valic acid residue) (Non-patent Document 8). However, the depsipeptide unit is not considered to be a material which exerts temperature responsibility in an aqueous solvent containing no organic solvent. This is because a related compound Boc-(Gly-Val-Gly-Hmb-Pro)ₙ-OBzl (n = 1 to 3) (Boc = t-butoxycarbonyl, OBzl = benzyl ester) which has been reported in the same document has a short length and has protective groups at both ends, so it cannot be made into an aqueous solution without adding 2.5% or more hexafluoroisopropanol, and the temperature responsibility has not been observed visually or by turbidity in the aqueous solution. Accordingly, the depsipeptide unit -Gly-X₂-Cly-Hmb-Pro- (X₂ = any α-amino acid residue) is thought to be unsuitable as a temperature responsive material and thus has not been investigated intensively.
Patent Document 1 JP 07-228639 A
Patent Document 2: JP 08-143631 A
Patent Document 3: JP 09-169850 A
Patent Document 4: JP 10-273451 A
Patent Document 5: JP 2000-212144 A
Patent Document 6: JP 2000-344834 A
Patent Document 7: JP 2002-256075 A
Patent Document 8: JP 2003-252936 A
Patent Document 9: JP 11-322941 A
Patent Document 10: JP 2000-80158 A
Patent Document 11: JP 2003-160473 A
Patent Document 12: JP 2004-35791 A
Patent Document 13: JP 2002-256075 A
Patent Document 14: JP 2001-514263 A
Patent Document 15: JP 2004-501784
Non-patent Document 1: Journal of Organic Chemistry 1985, Vol. 50, p715
Non-patent Document 2: Journal of Biomedical Materials Research, 1977, Vol. 11, p405
Non-patent Document 3: Journal of Macromolecular Science-Chemistry, 1984, Vol. A21, p561
Non-patent Document 4: Macromolecular Chemie, 1983, Vol. 184, p1761
Non-patent Document 5: Biomaterials, 1989, Vol. 10, p569
Non-patent Document 6: Journal of Biomedical Materials Research, 1990, Vol.24, p1173
Non-patent Document 7: Progress in Biophysics and Molecular Biology, 1992, Vol. 57, p23
Non-patent Document 8: Peptide Science 2003, The Japanese Peptide Society, 2004, p177

### DISCLOSURE OF THE INVENTION

It is an object of the present invention to provide a novel material obtained by combining a depsipeptide which can be designed to have a wide spectrum of properties and temperature responsibility.

The inventors of the present invention have focused on a novel sequence to achieve the above-mentioned object. That is, the inventors of the present invention have focused on the sequence -Gly-Val-Gly-Val-Ala-Pro- (SEQ ID NO: 2) in elastin which have not attracted attention because of its insolubility, and introduced a substitution with valic acid to synthesize a depsipeptide -Gly-X₁-Gly-Hmb-Ala-Pro- (X₁ represents an arbitrary α-amino acid residue). First, Boc-(Gly-Val-Gly-Hmb-Ala-Pro)ₙ-OBzl (n = 1 to 6) was synthesized. It was confirmed that, in particular, a trimer to a hexamer (n = 3 to 6) of the sequence are easily made into an aqueous solution by removing a protective group at a carboxyl terminal or an amino terminal thereof, and exerts visually distinct temperature responsibility. Subsequently, derivatives such as a polymerized compound (for example, poly(Gly-Val-Gly-Hmb-Ala-Pro) and a copolymerized compound (for example, poly(Gly-Val-Gly-Hmb-Ala-Pro)-*co*-(Gly-Lys(Z)-Gly-Val-Ala-Pro)) were prepared and temperature responsibility thereof was confirmed.

Then, a compound having an initial depsipeptide unit -Gly-X₂-Gly-Hmb-Pro- (X₂ represents an arbitrary α-amino acid residue), such as poly(Gly-Val-Gly-Hmb-Pro)) was also investigated. As a result, the compound had water solubility different from that originally estimated, and it was found that the compound had temperature responsibility in an aqueous solvent. Then, related compounds such as poly(Gly-Thr-Gly-Hmb-Pro) and poly(Gly-Ile-Gly-Hmb-Pro) were synthesized. Extensive investigations have been made on various depsipeptides containing valic acid residues, and the present invention thus has been completed.

That is, according to the present invention, there is provided a linear depsipeptide polymer comprising a repeating unit selected from the group consisting of:
Gly-Val-Gly-Hmb-Ala-Pro
Gly-Val-Gly-Hmb-Pro
Gly-Thr-Gly-Hmb-Pro, and
Gly-Ile-Gly-Hmb-Pro
said linear depsipeptide polymer comprising 18 or more of amino acid residues and valic acid residues in total,
wherein Hmb represents a valic acid residue represented by the following formula (II) 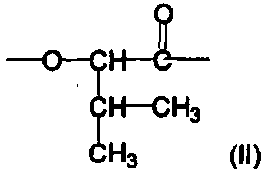 and wherein at least one of the amino acid residues at the N terminal and the C terminal of the linear depsipeptide polymer are deprotected.

In addition, the polymer compound of the present invention may be the above-mentioned polymer compound that has a terminal that is linked with a sugar chain sequence, a protein, a polysaccharide, a metal complex or a polymer carrier, gel, film, a latex particle, a metal fine particle, or a plastic plate. For example, the linear depsipeptide polymer which is poly[(Gly-Val-Gly-Hmb-Ala-Pro)-co-(Gly-Lys-Gly-Val-Ala-Pro)] is particularly preferable.

Further, according to the present invention, there is provided a temperature responsive composition comprising the above-mentioned polymer compounds.

### Brief Description of the Drawings

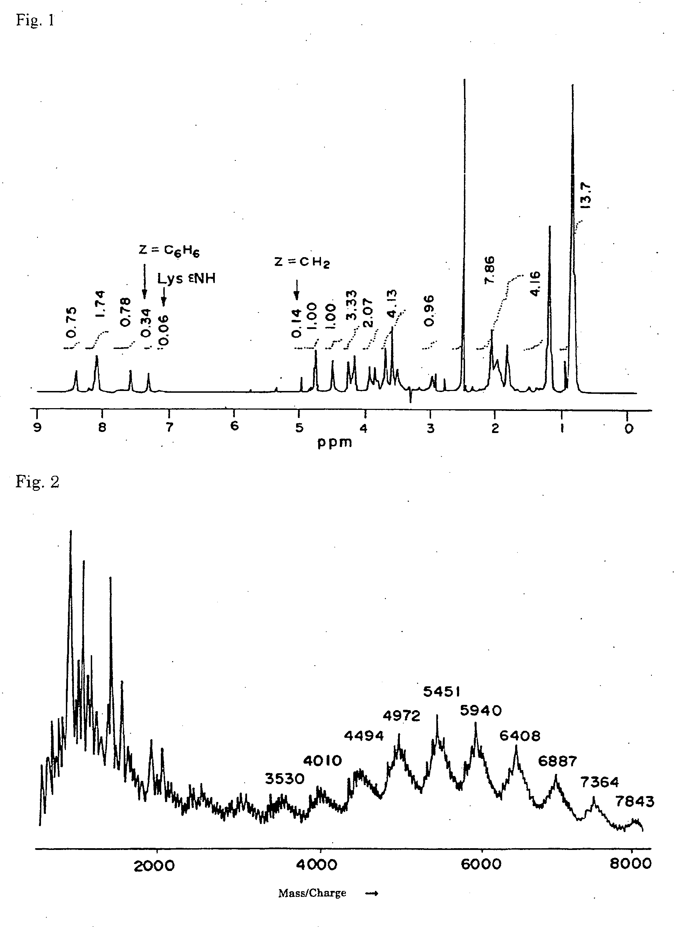

Fig. 1 shows a 500 MHz ¹H NMR spectrum of poly[Gly¹-Val²-Gly³-Hmb⁴-Ala⁵-Pro⁶]ₙ-*co*-(Gly⁷-Lys(Z)⁸-Gly⁹-Val¹⁰-Ala¹¹-pro¹²)₁₋ₙ], which is an embodiment of the present invention, measured in DMSO-d₆ at 30 °C.
Fig. 2 shows a spectrum of poly(Gly-Val-Gly-Hmb-Ala-Pro), which is an embodiment of the present invention, measured by MALDI-TOF mass spectrometry.

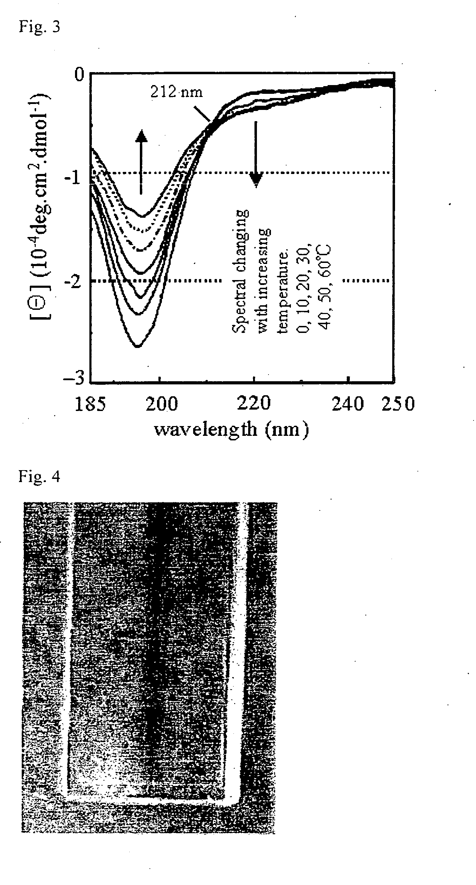

Fig. 3 shows circular dichroism spectra in terms of temperature of poly(Gly-Val-Gly-Hmb-Ala-Pro), which is an embodiment of the present invention, which are measured by: loading an aqueous solution of poly(Gly-Val-Gly-Hmb-Ala-Pro) (1 mg/1,000 µl) in a 1 mm-thick quartz cell for absorption spectrum; increasing temperature by 10 °C from 40 to 60 °C; and measuring the spectra at respective equilibrium temperatures.
Fig. 4 is a photograph of an aqueous solution of poly(Gly-Val-Gly-Hmb-Ala-Pro) (1 mg/50 µl), which is an embodiment of the present invention, loaded in a 1 mm-thick quartz cell for absorption spectrum and kept at 25°C.

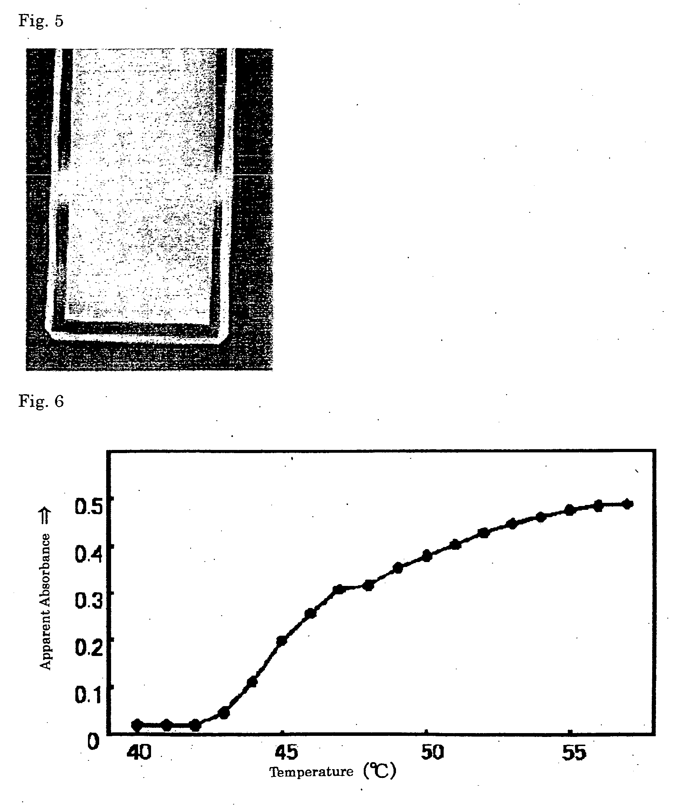

Fig. 5 is a photograph of an aqueous solution of poly(Gly-Val-Gly-Hmb-Ala-Pro) (1 mg/50 µl), which is an embodiment of the present invention, loaded in a 1 mm-thick quartz cell for absorption spectrum and kept at 55 °C.
Fig. 6 is a graph showing apparent absorbance of an aqueous solution of poly(Gly-Val-Gly-Hmb-Ala-Pro) (1 mg/50 µl), which is an embodiment of the present invention, at a wavelength of 500 nm which is measured by: loading the aqueous solution in a 1 mm-thick quartz cell for absorption spectrum; increasing temperature by 1 °C from 40 to 57 °C; and measuring and plotting the absorbance at respective equilibrium temperatures. The longitudinal axis represents the absorbance, and the lateral axis represents the temperature (°C).

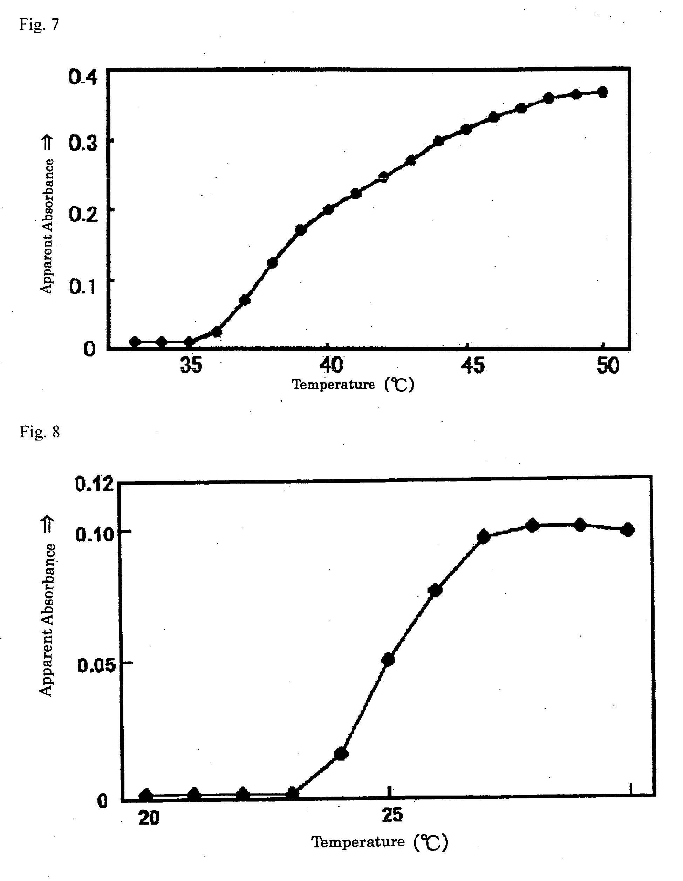

Fig. 7 is a graph showing apparent absorbance of an aqueous solution of poly[(Gly-Val-Gly-Hmb-Ala-Pro)-*co*-(Gly-Lys(Z)-Gly-Val-Ala-Pro)] (1 mg/50 µl), which is an embodiment of the present invention, at a wavelength of 500 nm which is measured by: loading the aqueous solution in a 1 mm-thick quartz cell for absorption spectrum; increasing temperature by 1 °C from 33 to 50 °C; and measuring and plotting the absorbance at respective equilibrium temperatures. The longitudinal axis represents the absorbance, and the lateral axis represents the temperature (°C).
Fig. 8 is a graph showing apparent absorbance of an aqueous solution of poly(Gly-Val-Gly-Hmb-Pro) (1 mg/50 µl), which is an embodiment of the present invention, at a wavelength of 500 nm which is measured by: loading the aqueous solution in a 1 mm-thick quartz cell for absorption spectrum; increasing temperature by 1 °C from 20 to 30 °C; and measuring and plotting the absorbance at respective equilibrium temperatures. The longitudinal axis represents the absorbance, and the lateral axis represents the temperature (°C).

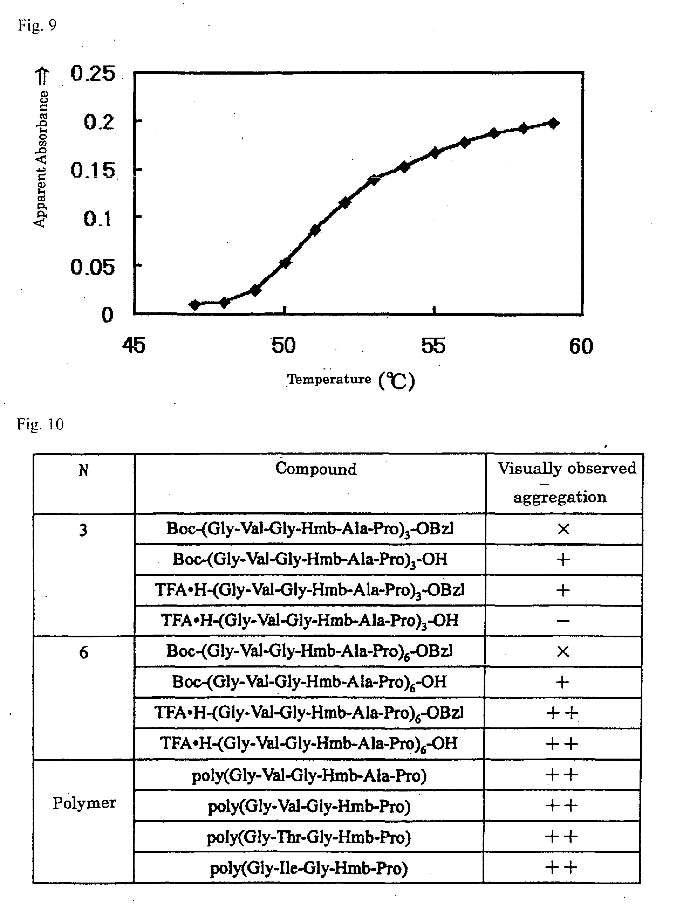

Fig. 9 is a graph showing apparent absorbance of an aqueous solution of TFA·H-(Gly-Val-Gly-Hmb-Pro)₅-OH (1 mg/50 µl), which is an embodiment of the present invention, at a wavelength of 500 nm which is measured by: loading the aqueous solution in a 1 mm-thick quartz cell for absorption spectrum; increasing temperature by 1 °C from 47 to 59 °C; and measuring and plotting the absorbance at respective equilibrium temperatures. The longitudinal axis represents the absorbance, and the lateral axis represents the temperature (°C).
Fig. 10 shows aggregation of aqueous solutions (concentration: 1.0 mg/20 µl) of X-(Gly-Val-Gly-Hmb-Ala-Pro)ₙ-Y which is an embodiment of the present invention, which is observed by loading each of the aqueous solutions in a glass tube having a diameter of 5 mm and heating each of the aqueous solutions at 80°C. "++" denotes strong aggregation, "+" denotes aggregation, "-" denotes no aggregation, and "x" denotes failure of test because of water insolubility.

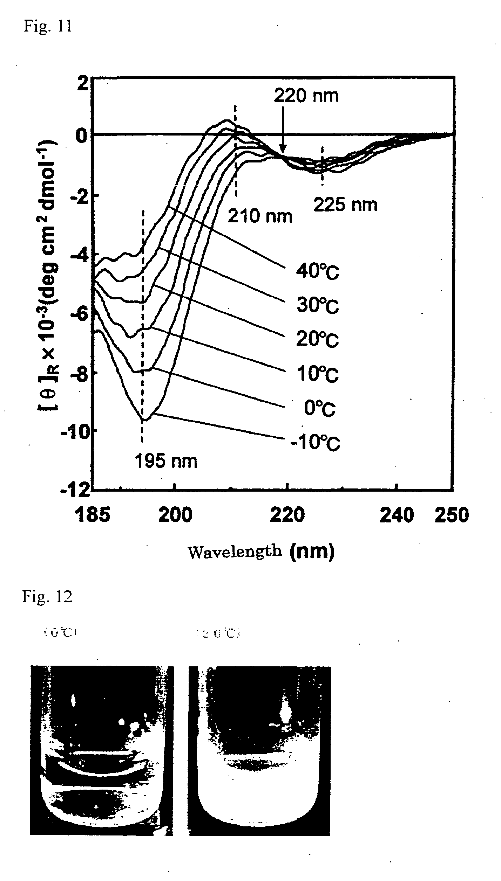

Fig. 11 shows circular dichroism spectra in terms of temperature of poly(Gly-Ile-Gly-Hmb-Pro) which is an embodiment of the present invention, which are measured by: loading an aqueous solution of poly(Gly-Ile-Gly-Hmb-Pro) (0.1 mg/1,000 µl) in a 1 mm-thick quartz cell for absorption spectrum; increasing temperature by 10°C from -10 to 40°C; and measuring the spectra at respective equilibrium temperatures.
Fig. 12 shows photographs of an aqueous solution of poly(Gly-Val-Gly-Hmb-Ala-Pro) which is an embodiment of the present invention, loaded in a 1 mm-thick quartz cell for absorption spectrum and kept at 0 °C and 20 °C.

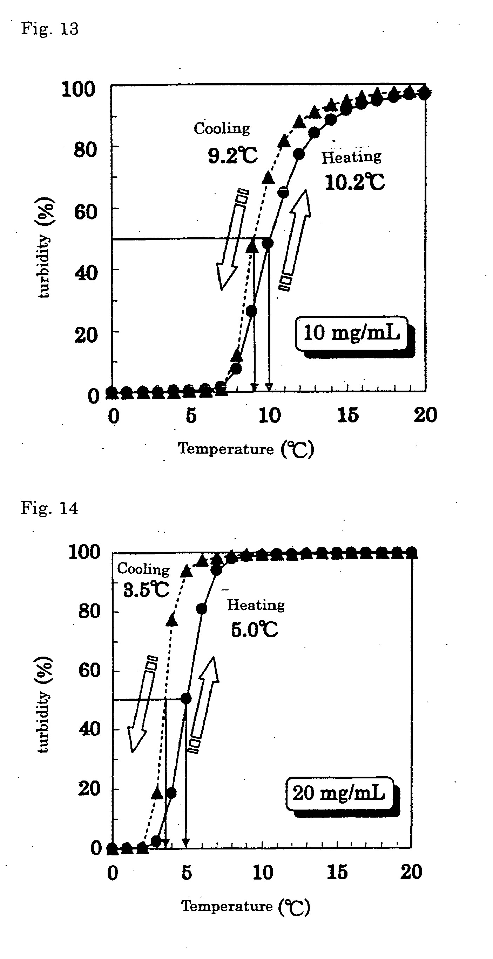

Fig. 13 shows transmittance (%) of poly(Gly-Val-Gly-Hmb-Ala-Pro) which is an embodiment of the present invention at a wavelength of 500 nm, which is measured by: loading an aqueous solution of poly(Gly-Val-Gly-Hmb-Ala-Pro) (10 mg/1,000 µl) in a 1 mm-thick quartz cell for absorption spectrum; increasing or decreasing temperature by 1°C between 0 to 20°C; and measuring the transmittance at respective equilibrium temperatures. In the graph, the longitudinal axis represents turbidity (100 - transmittance (%)), and the lateral axis represents temperature (°C).
Fig. 14 shows transmittance (%) of poly(Gly-Val-Gly-Hmb-Ala-Pro) which is an embodiment of the present invention at a wavelength of 500 nm, which is measured by: loading an aqueous solution of poly(Gly-Val-Gly-Hmb-Ala-Pro) (20 mg/1,000 µl) in a 1 mm-thick quartz cell for absorption spectrum; increasing or decreasing temperature by 1 °C between 0 to 20°C; and measuring the transmittance at respective equilibrium temperatures.
In the graph, the longitudinal axis represents turbidity (100 - transmittance (%)), and the lateral axis represents temperature (°C).

### DESCRIPTION OF THE PREFERRED EMBODIMENTS

The compound of the present invention is a linear depsipeptide polymer comprising a repeating unit selected from the group consisting of:
Gly-Val-Gly-Hmb-Ala-Pro
Gly-Val-Gly-Hmb-Pro
Gly-Thr-Gly-Hmb-Pro, and
Gly-Ile-Gly-Hmb-Pro
said linear depsipeptide polymer comprising 18 or more of amino acid residues and valic acid residues in total,
wherein Hmb represents a valic acid residue represented by the following formula (II) 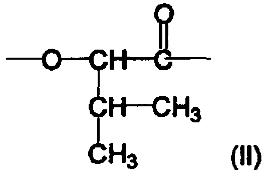 and wherein at least one of the amino acid residues at the N terminal and the C terminal of the linear depsipeptide polymer are deprotected.

The polymer compound of the present invention has a structure in which the structural units are repeated more than once. A number of repetitions is not particularly limited as long as the compound contains 18 or more of amino acid residues and hydroxy acid residues in total. The compound may be an oligomer having about 2 to 10 repetitions. A total number of the amino acid residues and the valic acid residues contained in the polymer compound of the present invention is preferably 1,000 or less, and more preferably 500 or less. The polymer compound of the present invention preferably has a molecular weight of 1,500 to 100,000, and more preferably 1,500 to 50,000. The polymer compound of the present invention having deprotected at least one of the amino acid residues at the N terminal and the C terminal is suitable for being dissolved in an aqueous environment.
The polymer compound of the present invention may comprise 2 or more kinds of the repeating unit selected from the group consisting of:
Gly-Val-Gly-Hmb-Ala-Pro
Gly-Val-Gly-Hmb-Pro
Gly-Thr-Gly-Hmb-Pro, and
Gly-Ile-Gly-Hmb-Pro,
or may include a repeating unit selected from this group and another repeating unit such as a polypeptide.

The polymer compound of the present invention can be obtained by, for example, synthesizing a compound (depsipeptide) having the repeating unit and then polymerizing the compound. The polymerization can be performed by general amide condensation reaction, but when the compound contains an amino acid having a reactive side chain, it is preferable to protect the side chain before the polymerization reaction.
In addition, so-called segment condensation, that is, elongation reaction in which the units are elongated one by one may be used.

The polymer compound of the present invention may have a terminal that is linked with another compound or material. The term "terminal" may refer to any one of the amino terminal and the carboxyl terminal or to both of them.
Examples of the another compound or material include a hydroxy acid sequence, an amino acid sequence, a sugar chain sequence, a protein, a polysaccharide, a metal complex or a polymer carrier, gel, a film, a latex particle, a metal fine particle, and a plastic plate. The polymer compound of the present invention can be linked with the another compound or material by a covalent bond, a coordination bond, an ionic bond, a hydrophobic interaction, a hydrogen bond, or the like. For example, the linear depsipeptide polymer which is poly[(Gly-Val-Gly-Hmb-Ala-Pro)-*co*-(Gly-Lys-Gly-Val-Ala-Pro)] is particularly preferable.

The compound of the present invention preferably has temperature responsibility.
The term "temperature responsibility" refers to a property of aggregating in water or a buffer solution when the compound is heated from a lower first temperature to a higher second temperature. The first temperature and the second temperature vary depending on the kind of the amino acid contained in the polymer compound or the kind of the another compound to be linked to the terminal of the polymer compound, and are appropriately selected depending on the polymer compound. It is preferable that there is a difference of 10 °C or more between the first temperature and the second temperature.
For example, in a case of a polymer compound whose response is slow, the difference between the first temperature and the second temperature requires to be about 30 °C. As shown in Figs. 4 and 5, poly(Gly-Val-Gly-Hmb-Ala-Pro) is observed to aggregate by being heated from 25 °C to 55 °C.
In addition, for example, in a case of a polymer compound whose response is rapid, the difference between the first temperature and the second temperature may be about 10 to 20 °C. As shown in Fig. 12, poly(Gly-Val-Gly-Hmb-Ala-Pro) is observed to aggregate by being heated from 0°C to 20°C.
The aggregation can be confirmed visually, or by a change in transmittance measured by a spectrometer, or by an apparent change in absorbance.
Since the polymer compound of the present invention has the above-mentioned properties, it can be used for production of a temperature responsive composition.

In the temperature responsive composition, the polymer compound of the present invention or the polymer compound of the present invention being linked with the another compound or material can be used alone, or in combination with a physiologically-acceptable carrier.

The physiologically-acceptable carrier is not particularly limited, and may be a solid agent such as powder or powdered drug. However, the polymer compound of the present invention is generally used as a liquid composition by being combined with a liquid carrier. That is, examples of the carrier for a liquid composition include: water; physiological saline; a buffer solution such as phosphate buffered solution; an aqueous alcohol solution such as an aqueous ethanol solution; an aqueous polyalcohol solution such as an aqueous 5% glycerin solution, an aqueous ethylene glycol solution, or an aqueous propylene glycol solution; an aqueous sugar solution such as an aqueous 5% glucose solution or an aqueous glucose solution; and an aqueous albumin solution such as an aqueous 5% albumin solution. The liquid composition may be a solution, a suspension, an emulsion, an ointment, an aerosol, a patch (paste or cataplasms), or the like. The liquid composition contains the temperature responsive material (or temperature responsive polymer) in a concentration selected from, for example, about 0.1 to 90% by weight, preferably about 0.5 to 50% by weight, more preferably about 1 to 30% by weight (for example, 1 to 15% by weight), and particularly preferably about 1 to 10% by weight, depending on a solution viscosity of the polymer or the like.

The temperature responsive composition may contain various physiologically-acceptable or pharmacologically-acceptable additives such as polymers such as cellulose derivatives (cellulose ethers and the like), polyvinyl pyrrolidone, macrogol, and polyvinyl alcohol, preservatives, stabilizers, emulsifiers, suspending agents, pH adjusters, buffering agents, and drugs (bactericides, disinfectants, antibacterial agents, antiviral agents, anti-inflammatory agents, antiallergic agents, painkillers, hemostatic drugs, and the like).

The temperature responsive composition of the present invention has a property of aggregating in response to temperature (for example, changing from a liquid to gel), and has an advantage that it is highly safe in living bodies. Thus, the temperature responsive composition of the present invention can be applied to the living bodies (for example, an affected area) to form a gel-like film on the applied area. The polymer and the composition of the present invention can be utilized for constituting compositions which are decomposed and absorbed in living bodies, compositions which are decomposed and absorbed in environment such as soil, cell adhesives, wound-covering materials, microcapsules, biological machines, biosensors, separation membranes, test kits, and the like. These can easily be developed on the basis of the related investigations of the inventors of the present invention (for example, Yoshida et al., Advanced materials, 1997, vol. 9. pp. 757; Hiroki et al., Journal of Polymer Science, 1998, vol. 36, pp. 1495; JP 7-233194 A; Yoshida et al., Drug Design and Delivery, 1991, vol. 7, pp. 159; Mashita et al., Kitakanto Medical Journal, 1991, vol. 41, pp. 311).

### Examples

Hereinafter, a method of synthesizing a -Gly-Val-Gly-Hmb-Ala-Pro- unit which is an embodiment of the present invention, and methods of synthesizing a dimer to a hexamer, that is, -(Gly-Val-Gly-Hmb-Ala-Pro)ₙ- (n = 1 to 6), a polymerized compound poly(Gly-Val-Gly-Hmb-Ala-Pro), and a copolymerized compound poly(Gly-Val-Gly-Hmb-Ala-Pro)-*co*-(Gly-Lys(Z)-Gly-Val-Ala-Pro)), which are obtained by using the -Gly-Val-Gly-Hmb-Ala-Pro-unit, will be described in detail in Examples 1 to 4, respectively. In addition, synthesis of poly(Gly-Val-Gly-Hmb-Pro), poly(Gly-Thr-Gly-Hmb-Pro), and poly(Gly-Ile-Gly-Hmb-Pro) as other embodiments will be described in detail in Examples 5 to 7, respectively. In addition, procedures commonly used in the Examples will be described in Synthesis Procedures 1 to 3. However, the present invention is not limited to the following specific examples, and it is needless to say that the modifications such as replacement of a protective group or condensation agent with other conventional ones can be appropriately made.

In the another depsipeptide unit, for example, -Gly-X₁-Gly-Hmb-Pro- or -Gly-X₂-Gly-Hmb-Pro-, an arbitrary amino acid residue is introduced into each of X₁ and X₂ in the same manner by using a corresponding N^{α}-t-butoxycarbonyl-amino acid instead of N^{α}-t-butoxycarbonyl-L-valine used in the examples.

Following abbreviations are used in the following examples.

### (amino-acid derivatives)

Boc-Gly-OH = *N*^{α}-*t*-butoxycarbonyl-glycine
Boc-Ala-OH = *N*^{α}-*t*-butoxycarbonyl-L-alanine
Boc-Val-OH = *N*^{α}-*t*-butoxycarbonyl-L-valine
Boc-Thr-OH = *N*^{α}-*t*-butoxycarbonyl-L-threonine
Boc-Lys(Z)-OH = *N*^{α}-*t*-butoxycarbonyl-*N*^{ε}-benzyloxycarbonyl-L-lysine
HCl·H-Pro-OBzl = L-proline benzyl ester hydrochloride

### (Main chain or side chain protective groups of amino acids)

Boc = *tert*-butoxycarbonyl (*t*-Bu-O-CO-)
OBzl = benzyl (-O-CH-₂-C₆H₅)

### (Reagents for peptide synthesis and related compounds)

DCC = *N,N*'-dichlorohexylcarbodiimide
DCUrea = dichlorohexylurea
HOSu = *N*-hydroxysuccinimide
HOBt = 1-hydroxybenzotriazole
TFA = trifluoroacetic acid
(Boc)₂O = di-*t*-butylcarbonate
NMM = *N*'-methylmorpholine
EDC·HCl = 1-ethyl-3-(3-dimethylaminopropyl)-carbodiimide hydrochloridel 1
DMAP = *N,N'*-dimethylaminopyridine

### (Solvents)

THF = tetrahydrofuran
CHCl₃ = chloroform
CDCl₃ = deuterated chloroform
AcOEt = acetic ether
DMF = *N,N'*-dimethylformamide
DMSO-*d*₆ = deuterated dimethylsulfoxide
MeOH = methanol
Et₂O = diethyl ether

### (Other)

TLC = thin layer chromatography

### (Synthesis Procedure 1: Synthesis of Boc-L-amino acid)

An L-amino acid or an L-amino acid having a protected side chain (1.0 mol) was dissolved in 4 M NaOH (250 ml, 1.0 mol), and gradually added with (Boc)₂O (240.0 g, 1.1 mol) which had been dissolved in advance in a minimum amount of dioxane while being gradually cooled by ice-cold MeOH over 30 minutes. The mixture was stirred in an ice bath for 1 hour and then at room temperature for 1.5 hours. Precipitated Na₂CO₃ was filtrated out, followed by drying with Na₂SO₄. After filtration, the filtrate was concentrated under reduced pressure, and a residue was subjected to crystallization using hexane. After that, recrystallization was performed by using AcOEt-hexane to obtain a Boc-L-amino acid.

### (Synthesis Procedure 2: Deprotection reaction of the amino-terminal and synthesis of a Boc-deprotected compound)

A peptide compound in which an amino group was protected by *N*^{α}-*t*-butoxycarbonyl was put in a 300-ml round-bottom flask, and added with TFA (or 4 M HCl/dioxane solution) to dissolve it in TFA (or 4 M HCl/dioxane solution) in a draft. Immediately after that, the round-bottom flask was sealed with a calcium chloride tube to prevent contamination of water. After termination of the reaction was confirmed by TLC, the reaction mixture was concentrated by repeatedly adding distilled Et₂O until a TFA smell (or hydrochloric acid smell) was eliminated, and thereby a white powder of a TFA salt (or hydrochloride) was finally obtained. The yield is substantially quantitative.

### (Synthesis Procedure 3: Condensation reaction)

A peptide compound (2.1 mmol) in which an amino group was protected by N-^{α}-t-butoxycarbonyl and a carboxyl terminal was deprotected was put in a 300-ml conical flask, dissolved in distilled CHCl₃, and added with HOBt (0.28 g, 2.1 mmol) and EDC·HCl (0.40 g, 2.1 mmol) (or DCC (0.43 g, 2.1 mmol)), followed by stirring. Next, the TFA salt of the peptide compound (1.4 mmol) in which the amino group had been deprotected by Synthesis Procedure 2 was put in a 300-ml round-bottom flask, and the TFA salt was neutralized with NMM. The neutralization can be achieved with a substantially equimolar amount (0.15 ml, 1.40 mmol) of NMM, but the amount of NMM sometimes increases in a case of a salt having bad crystallinity. The thus-prepared two solutions were mixed together and stirred, and immediately cooled with ice to initiate reaction. Then, it was gradually returned to room temperature and stirred overnight. After the mixture was concentrated by using an evaporator and dissolved in AcOEt (in a case where DCC was used, DCUrea insoluble in AcOEt was removed), it was washed sequentially with 10% citric acid, saturated NaHCO₃ distilled water, and saturated saline, then dried with Na₂S0₄, and concentrated to obtain an oil-like condensation product or a colorless powder of a condensation product. The condensation product was purified by silica gel chromatography (distilled CH₃Cl-petroleum ether) or gel filtration chromatography (LH20 manufactured by Pharmacia, DMF or MeOH). In a case of the colorless powder, it may be purified by recrystallization using a solvent system of AcOEt-distilled Et₂O or distilled CH₃Cl-petroleum ether. The yield is about 70 to 90%.

### Example 1

### (1) Synthesis of Boc-Gly-Val-Gly-Hmb-Ala-Pro-OBzl

### (1a: Synthesis of valic acid)

Valine (835 g, 0.5 mol) was put in a 1-L three-necked round bottom flask, and 500 ml of distilled water containing 1 M H₂SO₄ (18 M H₂SO₄, 27.7 ml) was added to dissolve valine. Next, the mixture was added with 2 equivalents of a saturate aqueous sodium nitrite solution (69.17 g) over 2 hours while being cooled with ice and stirred at 0°C or lower. After stirring until no foam was generated, it was subjected to extraction with Et₂O (about I L), and dried with NaHCO₃ and concentrated to obtain a white crystal.
Yield, 45.7 g (77.5%). ¹H NMR (CDCl₃, 300 MHz): 4.15 (1H, Hmb αCH); 2.09 (1H, Hmb βCH); 1.50, 1.00 (6H, Hmb γCH₃).

### (1b: Synthesis of Boc-Gly-OSu)

Boc-Gly-OH (1.54 g, 8.82 mmol) was put in a 500-ml round-bottom flask, dissolved in distilled CHCl₃, and added with DCU (2.18 g, 10.58 mmol) and HOSu (1.21 g, 10.58 mmol), followed by stirring under cooling. After the mixture was left overnight, termination of the reaction was confirmed by TLC. Then, the reaction mixture was concentrated, and DCUrea was filtrated out. The filtrate was dissolved in AcOEt, and DCUrea precipitated again was filtrated out. After that, the resultant filtrate was concentrated and subjected to recrystallization using AcOEt-distilled Et₂O twice to obtain Boc-Gly-OSu.
Yield, 1.74 g (63.9%). ¹H NMR (CDCl₃; 300 MHz): 5.04 (1H, Boc-Gly NH); 4.27, 4.23 (2H, Gly αCH₂); 2.79 (4H. OSu); 1.40 (9H, Boc *t*-Bu).

### (1c: Synthesis of Boc-Gly-Hmb-OH)

Boc-Gly-OSu (1.00 g, 3.7 mmol) was put in a 500-ml round-bottom flask and dissolved in distilled THF, and DMAP (0.040 g, 0.37 mmol) was added thereto to be dissolved. Next, valic acid (0.52 g, 4.4 mmol) was put in a 300-ml round-bottom flask and dissolved in distilled THF, and added with pyridine (0.29 ml, 4.4 mmol) to protect a carboxyl terminal of the valic acid. The thus-prepared two solutions were mixed and stirred under cooling. After termination of the reaction was confirmed by TLC, the reaction mixture was concentrated and diluted by adding AcOEt. The mixture was washed with an aqueous 1 M HCl solution and distilled water, and then, the product was extracted with a saturated aqueous NaHCO₃ solution and pH was adjusted to 2 to 3 with an aqueous 2 M HCl solution. After that, extraction was performed again with AcOEt for solvent replacement. Then, the extract was washed with distilled water and a saturated aqueous NaCl solution, dried with Na₂CO₃, and concentrated to obtain a colorless oil-form product.
Yield, 1.1 g (substantially quantitative). ¹H NMR (CDCl₃, 300 MHz): 9.25 (1H, Hmb -COOH); 6.40, 5.12 (1H, Boc-Gly NH); 4.95 (1H, Hmb αCH); 4.13, 4.11 (1H, Gly αCH₂); 2.28 (1H, Hmb βCH); 1.50 (9H, Boc *t*-Bu); 1.02 (6H, Hmb γCH₃).

### (1d: Synthesis of HCl·H-Pro-OBzl)

Benzyl alcohol (100 ml) was put in a 1-L three-necked round-bottom flask in a ventilation food, and stirred under cooling. Thionyl chloride (50.0 g, 0.420 mol) was dropped thereto over 1 hour. Subsequently, proline (22.0 g, 0.190 mmol) was added thereto, and the mixture was slowly returned to room temperature and continuously stirred for 3 days. Benzyl alcohol was removed by using a rotary evaporator, followed by addition of distilled Et₂O for crystallization. Purification was performed by repeating recrystallization with hot ethanol.
Yield, 20.87 g (45.5%). ¹H NMR (CDCl₃, 300 MHz): 7.30 (5H, -OBzl C₆H₅); 5.21 (2H, OBzl -CH₂-); 4.80 (1H, Pro αCH); 3.51 (2H, Pro δCH₂); 2.20, 1.90 (2H, Pro βCH₂); 1.99 (2H, Pro γCH₂).

### (1e: Synthesis of Boc-Ala-Pro-OBzl)

Boc-Ala-OH (9.4 g, 50 mmol) was dissolved in distilled CHCl₃ and added with DCC (10g, 50 mmol). In another round-bottom flask, a distilled CHCl₃ solution of HCl·H-Pro-OBzl (10 g, 41 mmol) was prepared, and neutralized with NMM (4.5 ml, 41 mmol). Both of the solutions were mixed and stirred under cooling with ice to initiate condensation reaction. The mixture was slowly returned to room temperature and stirred overnight, and concentrated. The residue was added with EtOAc, and the precipitated DCUrea was repeatedly removed. The obtained solution was washed sequentially with 10% citric acid, distilled water saturated NaHCO₃ distilled water, and saturated saline, dried with Na₂SO₄ and concentrated to obtain a colorless powder. The colorless powder was purified by recrystallization using AcOEt-distilled Et₂O.
Yield, 13.2 g (85%). ¹H NMR (CDCl₃, 300 MHz): 7.33 (5H, -OBzl C₆H₅); 5.36 (1H, Ala NH); 5.14 (2H, OBzl -CH₂-); 4.58 (1H, Pro αCH); 4.45 (1H, Ala αCH); 3.60 (2H. Pro δCH₂); 2.20, 1.90 (2H, Pro βCH₂); 1.99 (2H, Pro γCH₂); 1.41 (9H, Boc *t*-Bu); 1.28 (3H, Ala βCH₂).

### (1f: Synthesis of Boc-Gly-Hmb-Ala-Pro-OBzl)

Condensation reaction was performed by using Boc-Gly-Hmb-OH (4.59 g, 16.6 mmol), HOBt (2.25 g, 16.6 mmol), EDC·HCl (3.17 g, 16.6 mmol), HCl·H-Ala-Pro-OBzl (4.15 g, 13.34 mmol), and NMM (1.46 ml, 13.34 mmol). After that, purification was performed by silica gel chromatography using a developing solvent of a mixture of CHCl₃ and MeOH (CHCl₃:MeOH = 97:3) to obtain an oil-form target product.
Yield, 3.91 g (54.9%). ¹H NMR (CDCl₃, 300 MHz): 7.35, 7.14 (2H, Ala NH, Gly NH); 7.35 (5H, -OBzl, C₆H₅); 5.15 (2H, -OBzl, -CH₂-); 5.08 (Hmb αCH); 4.70 (1H, Ala αCH); 4.57 (1H, Pro αCH); 4.02 (2H, Gly αCH₂); 2.27, 2.00 (2H, Pro βCH₂); 2.00 (1H, Val βCH); 1.35 (3H, Ala βCH₂); 1.45 (9H, Boc t-Bu); 0.93 (6H, Val γCH₃); 2.00 (2H, Pro γCH₂); 3.65 (2H, Pro δCH₂), 0.98 (9H, Boc *t-*Bu).

### (1g: Synthesis of Boc-Val-Gly-Hmb-Ala-Pro-OBzl)

Condensation reaction was performed by using Boc-Val-OH (0.52 g, 2.41 mmol), HOBt (0.32 g, 2.41 mmol), EDC·HCl (0.46 g, 2.41 mmol), TFA·H-Gly-Hmb-Ala-Pro-OBzl (1.02 g, 1.87 mmol), and NMM (0.20 ml, 1.87 mmol) to obtain an oil-form product.
Yield, 1.18 g (77.1%). ¹H NMR (CDCl₃, 300 MHz): 7.34 (5H, -OBzl C₆H₅); 7.12 (1H, Ala NH); 6.80 (1H, Gly NH); 5.38 (1H, Val NH); 5.15 (2H, -OBzl -CH₂-); 5.07 (1H, Hmb αCH); 4.72 (1H, Ala αH); 4.61 (1H, Pro αCH); 4.21 (1H, Val αCH); 4.05 (2H, Gly αCH₂); 2.21 (2H, Pro βCH₂); 2.09 (1H, Hmb βCH); 1.95 (1H, Val βCH); 1.29 (3H, Ala βCH₃); 2.00 (2H, Pro δCH₂); 0.90, 0.81 (12H, Val γCH₃, Hmb γCH₃); 3.64, 3.50 (2H, Pro δCH₂); 0.98 (9H, Boc *t*-Bu).

### (1h: Synthesis of Boc-Gly¹-Val²-Gl³-Hmb⁴-Ala⁵-Pro⁶-OBzl)

Condensation reaction was performed by using Boc-Gly-OH (0.369 g, 2.11 mmol), HOBt (0.284 g, 2.11 mmol), EDC-HCl (0.40 g, 2.11 mmol), TFA·H-Val-Gly-Hmb-Ala-Pro-OBzl (0.90 g, 1.40 mmol), and NMM (0.15 ml, 1.40 mmol). Purification was performed by gel filtration chromatography.
Yield, 0.39 g (40%, oil-like product). ¹H NMR (DMSO-*d*₆, 300 MHz): 8.47 (1H, Gly³ NH); 8.14 (1H, Ala⁵ NH); 7.63 (1H, Val² NH); 6.98 (1H, Gly NH); 7.34 (5H, -OBzl C₆H₅); 5.09 (2H, -OBzl -CH₂-); 4.76 (1H, Hmb⁴ αCH); 4.50 (1H, Ala⁵ αCH); 4.33 (1H, Pro⁶ αCH); 4.21 (1H, Val² αCH); 3.90, 3.58 (4H, Gly¹ αCH₂, Gly³ αCH₂); 2.20 (2H, Pro⁶ βCH₂); 2.10, 2.00 (2H, Val² βCH, Hmb⁴ βCH); 1.18 (3H, Ala⁵ βCH₃); 2.00 (2H, Pro⁶ γCH₂); 0.86 (12H, Val² γCH₃, Hmb⁴ γCH₃); 3.56 (2H, Pro⁶ δCH₂).

### Example 2

### (2) Synthesis of Boc-(Gly¹-Val²-Gly³-Hmb⁴-Ala⁵-Pro⁶)ₙ-OBzl (n = 2 to 6)

### (2a: Synthesis of Boc-Gly¹-Val²-Gly³-Hmb⁴-Ala⁵-Pro⁶-OH)

Boc-Gly¹-Val²-Gly³-Hmb⁴-Ala⁵-Pro⁶-OBzl (1.14 g, 1.65 mmol) was put in a 500-ml round-bottom flask and dissolved in MeOH, and added with palladium carbon powder. After an apparatus was built, the round-bottom flask was filled with H₂, and the mixture was stirred to initiate catalytic reduction reaction. Progression of the reaction was confirmed by elevation of a liquid surface, and the termination of the reaction was determined when spots of the raw materials in TLC were eliminated. After that, the palladium carbon powder was removed, and the filtrate was concentrated, followed by azeotropy with benzene. The residue was added with distilled CH₃Cl-petroleum ether for crystallization.
Yield, 9.28 g (93%); [α]_{D}²⁰ = -87.4 deg. (MeOH, c 0.1), melting point = 117-119 °C. ¹H NMR (DMSO-d₆, 300 MHz): 12.39 (1H, OH); 8.51 (1H, Gly³·H); 8.14 (1H, Ala⁵·H); 7.63 (1H, Val²·H); 6.99 (1H, Boc-Gly¹ NH); 4.77 (1H, Hmb⁴ αCH); 4.49 (1H, Ala⁵ αCH); 4.21 (2H, Pro⁶ αCH, Val² αCH); 3.89, 3.60 (4H, Gly¹ αCH₂, Gly³ αCH₂); 2.00 (4H, Pro⁶-γ, βCH₂); 1.18 (2H, Val² βCH, Hmb⁴ βCH); 1.37 (9H, Boc *t-*Bu); 0.85 (12H, Val² γCH₃, Hmb⁴ γCH₃); 3.50 (2H, Pro⁶ δCH₂).

### (2b: Synthesis of Boc-(Gly¹-Val²-Gly³-Hmb⁴-Ala⁵-Pro⁶)₂-OBzl)

Condensation reaction was performed by using TFA·H-Gly¹-Val²-Gl³-Hmb⁴-Ala⁵-Pro⁶-OBzl (0.28 g, 0.41 mmol), NMM (0.04 ml. 0.41 mmol), Boc-Gly¹-Val²-Gly³-Hmb⁴-Ala⁵-Pro⁶-OH (0.25 g, 0.41 mmol), EDC-HCl (0.09 g, 0.41 mmol), and HOBt (0.06 g, 0.41 mmol). Purification was performed by repeating recrystallization in a solvent system of distilled CHCl₃-distilled Et₂O.
Yield, 0.37 g (75.8%); [α]_{D}²⁰ = -101.2 deg. (MeOH, c 0.1), melting point = 123-126 °C. ¹H NMR (DMSO-d₆. 300 MHz): 8.40 (2H, Gly³ NH); 8.14 (3H, Ala⁵NH, Gly¹ NH); 7.61 (2H, Val² NH); 6.99 (1H, Boc-Gly¹ NH); 7.33 (5H. -OBzl C₆H₅); 5.09 (2H, -OBzl -CH₂-); 4.77 (2H, Hmb⁴ αCH); 4.50 (2H, Ala⁵ αCH); 4.38, 4.28 (2H, Pro⁶ αCH); 4.23 (2H, Val² αCH); 3.92, 3.52 (8H, Gly¹ αCH₂, Gly³ αCH₂); 2.09 (1H, Hmb⁴ βCH); 1.86 (1H, Val² βCH); 2.18, 1.95 (2H, Pro⁶ βCH₂); 1.19 (6H, Ala⁵ βCH₃); 1.17 (9H, Boc *t*-Bu); 0.86 (24H, Val² γCH₃, Hmb⁴ γCH₃); 1.98 (4H, Pro⁶ γCH₂); 3.57 (4H, Pro⁶ δCH₂).

### (2c: Synthesis of Boc-(Gly¹-Val²-Gl³-Hmb⁴-Ala⁵-Pro⁶)₃-OBzl)

Condensation reaction was performed by using TFA·H-(Gly¹-Val²-Gly³-Hmb⁴-Ala⁵-Pro⁶)₂-OBzl (0.33 g, 0.28 mmol), NMM (0.03 ml, 0.28 mmol), Boc-Gly¹-Val²-Gly³-Hmb⁴-Ala⁵-Pro⁶-OH (0.16 g, 0.28 mmol), EDC-HCl (0.04 g, 0.27 mmol), and HOBt (0.03 g, 0.28 mmol). Purification was performed by gel filtration chromatography. Yield: 0.37 g (80%), [α]_{D}²⁰: -110.6 deg. (MeOH, c 0.1), melting point: 152-154°C. ¹H NMR (DMSO-*d*₆,300 MHz): 8.48 (3H, Gly³ NH); 8.12 (5H, Ala⁵, Gly³ NH); 7.62 (3H, Val² NH); 6.99 (1H, Boc-Gly¹NH); 7.34 (5H, -OBzl C₆H₅); 5.09 (2H, -OBzl -CH₂-); 4.77 (3H, Hmb⁴ aCH); 4.51 (3H, Ala⁵ αCH); 4.29, 4.20 (3H, Pro⁶ αCH); 4.23 (3H, Val² αCH); 3.91, 3.70 (12H, Gly¹ αCH₂, Gly³ αCH₂); 2.00, 2.20 (6H, Pro⁶ βCH₂); 1.85, 2.00 (6H, Val² βCH, Hmb βCH); 1.37 (9H, Boc *t*-Bu); 2.05 (6H, Pro⁶ γCH₂); 0.89 (36H, Val², Val⁴, Hmb γCH₃); 3.58 (6H, Pro⁶ δCH₂).

### (2d: Synthesis of Boc-(Gly¹-Val²-Gly³-Hmb⁴-Ala⁵-Pro⁶)₄-OBzl)

Condensation reaction was performed by using TFA·H-(Gly¹-Val²-Gly³-Hmb⁴-Ala⁵-Pro⁶)₃-OBzl (0.34 g, 0.20 mmol), NMM (0.02 ml, 0.20 mmol), Boc-Gly¹-Val²-Gly³-Hmb⁴-Ala⁵-Pro⁶-OH (0.12 g, 0.30 mmol), EDC·HCl (0.05 g, 0.30 mmol), and HOBt (0.04 g, 0.30 mmol). Purification was performed by gel filtration chromatography.
Yield = 0.34 g, 77%, [α]_{D}²⁰ = -114.2 deg. (MeOH, c 0.1); melting point = 150-156 °C. ¹H NMR (DMSO-*d*₆, 300 MHz): 8.46 (4H, Gly³ NH); 8.14 (7H, Ala⁵, Gly¹ NH); 7.63 (4H, Val² NH); 6.98 (1H, Boc-Gly¹ NH); 7.35 (5H, -OBzl, C₆H₅); 5.09 (2H, -OBzl, -CH₂-); 4.75 (4H, Hmb⁴ αCH); 4.50 (4H, Ala⁵ αCH); 4.29, 4.18 (4H, Pro⁶ αCH); 4.18 (4H, Val² αCH); 3.92, 3.71 (16H, Gly¹ αCH₂, Gly³ αCH₂); 2.0 (24H, Pro⁶ βCH₂, Pro⁶γCH₂, Val² βCH, Hmb⁴ βCH); 1.18 (12H, Ala⁵ βCH₃); 1.37 (9H, Boc *t*-Bu); 0.84 (48H, Val² γCH₃); 2.00 (8H, Pro⁶ δCH₃); 3.53 (8H, Pro⁶ δCH₂).

### (2e: Synthesis of Boc-(Gly¹-Val²-Gly³-Hmb⁴-Ala⁵-Pro⁶)₅-OBzl)

Condensation reaction was performed by using TFA·H-(Gly¹-Val²-Gly³-Hmb⁴-Ala⁵-Pro⁶)₄-OBzl (0.34 g, 0.15 mmol), NMM (17 µl, 0.15 mmol), Boc-Gly¹-Val²-Gly³-Hmb⁴-Ala⁵-Pro⁶-OH (0.11 g, 0.19 mmol), EDC·HCl (0.03 g, 0.19 mmol), and HOBt (0.02 g, 0.19 mmol). Purification was performed by gel filtration chromatography.
Yield, 0.28 g (67%); [α]_{D}²⁰ = -113.8; melting point = 159-162°C. ¹H NMR (DMSO-*d*₆, 300MHz): 8.45 (5H, Gly³ NH); 8.11 (9H. Ala⁵, Gly¹ NH); 7.60 (5H, Val² NH); 6.94 (1H, Boc-Gly¹ NH); 7.33 (5H, -OBzl -C₆H₅); 5.04 (2H, -OBzl -CH₂-); 4.75 (5H, Hmb⁴ αCH); 4.50 (5H, Ala⁵ αCH); 4.26 (5H, Pro⁶ αCH); 4.17 (5H, Val² αCH); 3.90, 3.69 (20H, Gly¹ αCH₂, Gly³ αCH₂); 2.00 (30H, Pro⁶ βCH₂, Pro⁶γCH₂, Val² βCH, Hmb⁴ βCH); 1.19 (15H, Ala⁵ βCH₃); 1.36 (9H, Boc *t*-Bu); 0.84 (60H, Val² γCH₃, Hmb⁴ γCH₃); 2.00 (10H, Pro⁶ γCH₂); 3.44 (10H, Pro⁶ δCH₂).

### (2f: Synthesis of Boc-(Gly¹-Val²-Gly³-Hmb⁴-Ala⁵-Pro⁶)₆-OBzl)

Condensation reaction was performed by using TFA·H-(Gly¹-Val²-Gly³-Hmb⁴-Ala⁵-Pro⁶)₅-OBzl (0.28 g, 0.10 mmol), NMM (11.7 µl, 0.10 mmol), Boc-Gly¹-Val²-Gly³-Hmb⁴-Ala⁵-Pro⁶-OH (0.07 g, 0.12 mmol), EDC·HCl (0.01 g, 0.12 mmol), and HOBt (0.01 g, 0.12 mmol). Purification was performed by gel filtration chromatography.
Yield, 0.10 g (33%); [α]_{D}²⁰= -120.1 deg. (MeOH, c 0.1); melting point = 164-166°C.

### Example 3

### (3) Synthesis of poly(Gly-Val-Gly-Hmb-Ala-Pro)

### (3a: Synthesis of Boc-Gly¹-Val²-Gly³-Hmb⁴-Ala⁵-Pro⁶-OSu)

Boc-Gly¹-Val²-Gly³-Hmb⁴-Ala⁵-Pro⁶-OH (0.38 g, 0.63 mmol) was dissolved in distilled DMF and added with DCC (0.14 g, 0.69 mmol) and HOSu (0.08 g, 0.69 mmol), and the reaction mixture was stirred under cooling with ice overnight. After that, termination of the reaction was confirmed by TLC, and DCUrea was removed, followed by concentration, thereby a white powder was obtained.
Yield, 0.35 g (80%). ¹H NMR (DMSO-*d*₆, 300 MHz): 8.50 (1H, Gly³ NH); 8.23 (1H, Ala⁵ NH); 7.63 (1H, Val²-NH); 6.99 (1H, Boc-Gly¹ NH); 4.78 (1H, Hmb⁴ αCH); 4.71 (1H, Pro⁶ αCH); 4.52 (1H, Ala⁵ αCH); 4.23 (1H, Val² αCH); 3.92, 3.60 (4H, Gly¹ αCH₂, Gly³ αCH₂); 2.79 (4H, -OSu); 2.00 (4H, Pro⁶ βCH₂, Val² βCH, Hmb⁴ βCH); 1.21 (3H, Ala⁵ βCH₃); 1.37 (9H, Boc *t*-Bu); 0.88 (12H, Val² γCH₃, Hmb⁴ γCH₃); 2.00 (2H, Pro⁶ γCH₂), 3.57 (2H, Pro⁶ δCH₂).

### (3b: Synthesis of TFA·H-Gly¹-Val²-Gly³-Hmb⁴-Ala⁵-Pro⁶-OSu)

The amino-terminal of Boc-Gly¹-Val²-Gly³-Hmb⁴-Ala⁵-Pro⁶-OSu (0.20 g, 0.28 mmol) was deprotected by using TFA to obtain a white powder.
Yield, 0.18 g (89%). ¹H NMR (DMSO-*d*₆, 300MHz): 8.60 (1H, Gly³ NH); 8.45 (1H, Ala⁵ NH); 8.25 (1H, Val² NH); 4.78 (1H, Hmb⁴ αCH); 4.70 (1H, Pro⁶ αCH); 4.51 (1H, Ala⁵ αCH); 4.29 (1H, Val² αCH); 3.95, 3.50 (4H, Gly¹ αCH₂, Gly³ αCH₂); 2.79 (4H, OSu); 2.00 (6H, Pro⁶ βCH₂, Pro⁶ γCH₂, Val² βCH, Hmb⁴ βCH); 1.21 (3H:Ala⁵ βCH₃); 0.88.(12H, Val² γCH₂, Hmb⁴ γCH²); 3.50 (2H, Pro⁶ δCH₂).

### (3c: Synthesis of poly(Gly¹-Val²-Gly³-Hmb⁴-Ala⁵-Pro⁶) by polymerization reaction in distilled DMF)

TFA·H-Gly¹-Val²-Gly³-Hmb⁴-Ala⁵-Pro⁶-OSu (0.18 g, 0.25 mmol) was put in a 100-ml round-bottom flask, dissolved in distilled DMF, and stirred. Tetraethylamine (139 µl, 0.25 mmol) was dropped thereto to initiate polymerization. After 2 weeks, the molecular weight of the product was confirmed by mass spectrometry at the time when the solution was gelated. For further polymerization, EDC·HCl (0.05 g, 0.25 mmol) and HOBt (0.03 g, 0.25 mmol) were added thereto, followed by stirring for another 2 weeks. After that, dialysis was performed for 1 week with a semipermeable membrane through which molecules having molecular weights of 3,500 or less can pass, and the dialysate was freeze-dried to obtain a target product.
Yield, 15 mg (8%); melting point = 172-180 °C.

### (3d: Synthesis of poly(Gly¹-Val²-Gly³-Hmb⁴-Ala⁵-Pro⁶) by polymerization reaction in distilled dioxane)

TFA·H-Gly¹-Val²-Gly³-Hmb⁴-Ala⁵-Pro⁶-OSu (0.80 g, 1.12 mmol) was put in a 100-ml round-bottom flask and dissolved in distilled dioxane, followed by stirring. Tetraethylamine (155 µl, 1.12 mmol) was dropped thereto to initiate polymerization. After 2 weeks, the molecular weight of the product was confirmed by mass spectrometry at the time when the solution was gelated. For further polymerization, the gel-like polymer was dissolved in distilled DMF, then EDC-HCl (0.21 g, 1.12 mmol) and HOBt (0.15 g, 1.12 mmol) were added thereto, followed by stirring for additional 2 weeks. After that, dialysis was performed for 1 week with a semipermeable membrane through which molecules having molecular weights of 3,500 or less can pass, and the dialysate was freeze-dried to obtain a colorless powder.
Yield = 91 mg (11%); melting point = 175-180°C; average molecular weight = 6,500 (apparent value obtained by MALDI-TOF mass spectrometry).

### Example 4

### (4) Synthesis of poly[(Gly¹-Val²-Gly³-Hmb⁴-Ala⁵-Pro⁶)-co-(Gly⁷-Lys(Z)⁸-Gly⁹-Val¹⁰-Ala¹¹-Pro¹²)]

### (4a: Synthesis of Boc-Lys(Z)-Gly-Val-Ala-Pro-OMe (SEQ ID NO: 3))

Condensation reaction was performed by using TFA·H-Gly-Val-Ala-Pro-OMe (SEQ ID NO: 4) (0.50 g, 1.06 mmol), NMM (substantially equimolar amount to that used for neutralization of a TFA salt), Boc-Lys(Z)-OH (0.48 g, 1.28 mmol), EDC·HCl (0.24 g, 1.28 mmol), and HOBt (0.17 g, 1.28 mmol). Crystallization was performed by using distilled CHCL₃-distilled Et₂O.
Yield = 0.50 g (65%); [α]_{D}²⁰ = -65.1 deg. (MeOH, c 0.1); melting point = 115-117°C. ¹H NMR (DMSO-*d*₆, 300 MHz): 8.15 (1H, Ala NH); 7.99 (1H, Lys(Z) εNH); 7.61 (1H, Val NH); 7.19 (1H, Gly NH); 6.86 (1H, Lys(Z) NH); 7.34 (5H, Z- C₆H₅); 4.98 (2H, Z -CH₂-); 4.46 (1H, Ala αCH); 4.27 (1H, Pro αCH); 4.18 (1H, Val αCH); 3.85 (1H, Lys(Z) αCH); 3.63 (2H, Gly αCH₂); 2.13, 1.92 (2H, Pro βCH₂); 2.13 (1H, Val βCH₂); 1.05 (3H, Ala βCH₃); 1.56 (2H, Lys(Z) βCH₂); 1.89 (2H, Pro γCH₂); 0.78 (6H, Val-γCH₃); 1.45 (2H, Lys(Z), γCH₂); 3.60 (2H, Pro δCH₂); 1.80 (2H, Lys(Z) δCH₂); 2.95 (2H, Lys(Z) εCH₂); 1.18 (3H, -OMe); 1.36 (9H. Boc *t*-Bu).

### (4b: Synthesis of Boc-Gly¹-Lys(Z)²-Gly³-Val⁴-Ala⁵-Pro⁶-OMe (SEQ ID NO: 5))

Condensation reaction was performed by using TFA·H-Lys(Z)-Gly-Val-Ala-Pro-OMe (SEQ ID NO: 6) (0.48 g. 0.69 mmol), NMM (82 µl. 0.72 mmol), Boc-Gly-OH (0.137 g, 0.785 mmol), EDC·HCl (0.149 g, 0.785 mmol), and HOBt (0.105 g, 0.785 mmol). Purification was performed by recrystallization using distilled CHCl₃-distilled Et₂O.
Yield, 0.33 mg (65%); [α]_{D}²⁰=-60.5 deg. (MeOH, c 0.1); melting point = 130-132°C. ¹H NMR (CDCl₃, 300 MHz): 8.29 (1H, Lys(Z)² εNH); 8.14 (1H, Ala⁵ NH); 7.87 (1H, Val⁴ NH); 7.62 (1H, Lys(Z)² NH); 7.18 (1H, Gly³ NH); 6.89 (1H, Boc-Gly¹ NH); 7.32 (5H, Z C₆H₅-); 4.98 (2H, Z -CH₂-); 4.46 (1H, Ala⁵ αCH); 4.29-4.16 (3H, Pro⁶ αCH, Val⁴ αCH, Lys(Z)² αCH), 3.70 (4H, Gly¹ αCH₂, Gly³ αCH₂); 2.17, 2.00 (2H, Pro⁶ βCH₂); 2.00 (1H, Val⁴ βCH₂); 1.17 (3H, Ala⁵ βCH₃); 1.62 (2H, Lys(Z)² βCH₂); 2.00 (2H, Pro⁶ γCH₂); 0.79 (6H, Val⁴γCH₃); 1.39 (2H, Lys(Z)² γCH₂); 3.60 (2H, Pro⁶ δCH₂); 1.80 (2H, Lys(Z)² δCH₂); 2.94 (2H, Lys(Z)² εCH₂); 1.18 (3H, -OMe); 1.36 (9H, Boc *t*-Bu).

### (4c: Synthesis of Boc-Gly¹-Lys(Z)²-Gly³-Val⁴-Ala⁵-Pro⁶-OH (SEQ ID NO: 7))

Boc-Gly¹-Lys(Z)²-Gly³-Val⁴-Ala⁵- Pro⁶-OMe (SEQ ID NO: 5) (0.33 g, 0.42 mmol) was put in a 100-ml round-bottom flask, dissolved in a mixed solution of MeOH and water, and added with an aqueous 1 M NaOH solution (0.637 ml), followed by stirring under cooling. After 1 hour, termination of the reaction was confirmed by TLC, and the solution was concentrated and washed with ether. After that, pH was adjusted to be around 3 to 4 by 1 N HCL, and the obtained solution was subjected to extraction with CHCL₃ and washed with a saturated aqueous NaCl solution, followed by drying with Na₂SO₄. Purification was performed by recrystallization using distilled CHCl₃-distilled Et₂O.
Yield, 0.27 mg (84%); [α]_{D}²⁰ = -63.1 deg. (MeOH, c 0.1); melting point = 105-109 °C. ¹H NMR (CDCl₃, 300 MHz): 8.19 (1H, Lys(Z)² εNH); 8.13 (1H, Ala⁵ NH); 7.87 (1H, Val⁴ NH); 7.62 (1H, Lys(Z)⁴ NH); 7.18 (1H, Gly³ NH); 6.89 (1H, Boc-Gly¹ NH); 7.34 (5H, Z- C₆H₅): 4.98 (2H, Z-CH₂), 4.46(1H, Ala⁵ αCH); 4.25-4.05 (3H, Pro⁶ αCH, Val⁴ αCH, Lys(Z)² αCH); 3.70 (4H, Gly¹ αCH₂, Gly³ αCH₂); 2.15, 1.90 (2H, Pro⁶ βCH₂); 1.90 (1H, Val⁴ βCH₂); 1.18 (3H, Ala⁵ βCH₃); 1.62 (2H, Lys(Z)² βCH₂); 1.90 (2H, Pro⁶ γCH₂); 0.77 (6H, Val⁴ γCH₃); 1.45 (2H, Lys(Z)² γCH₂); 3.60 (2H, Pro⁶ δCH₂); 1.80 (2H, Lys(Z)² δCH₂): 2.94 (2H, Lys(Z)² εCH₂), 1.36 (9H, Boc *t*-Bu).

### (4d: Synthesis of Boc-Glyl-Lys(Z)²-Gly³-Val⁴-Alas-Pro⁶-OSu (SEQ ID NO: 8))

Boc-Gly-Lys(Z)-Gly-Hmb-Ala- Pro-OH (SEQ ID NO: 7) (0.27 g, 0.35 mmol) was dissolved in distilled DMF and added with DCC (0.08 g, 0.42 mmol) and HOSu (0.05 g, 0.42 mmol), followed by stirring under cooling overnight. After that, termination of the reaction was confirmed by TLC, then DCUrea was removed, and the filtrate was concentrated to obtain a white powder.
Yield, 0.24 mg (80%). ¹H NMR (DMSO-*d*₆, 300 MHz): 8.25 (1H, Lys(Z)² εNH); 8.21 (1H. Ala⁵ NH); 7.86 (1H, Val⁴ NH); 7.63 (1H, Lys(Z)² NH); 7.19 (1H, Gly³ NH); 6.89 (1H, Boc-Gly¹ NH); 7.35 (5H, Z- C₆H₅); 4.98 (2H, Z- -CH₂-); 4.68 (1H, Ala⁵ αCH); 4.67 (1H, Pro⁶ αCH); (2H, Val⁴ αCH, Lys(Z)² αCH); 3.70 (4H, Gly¹ αCH₂, Gly³ αCH₂); 2.31, 1.90 (2H, Pro⁶ βCH₂); 1.90 (1H, Val⁴ βCH₂); 1.18 (3H, Ala⁵ βCH₃); 1.48 (2H, Lys(Z)² βCH₂); 1.90 (2H, Pro⁶ γCH₂); 0.78 (6H, Val⁴ γCH₃); 1.39 (2H, Lys(Z)² γCH₂); 3.55 (2H, Pro⁶ δCH₂); 1.35 (2H, Lys(Z)² δCH₂); 2.94 (2H, Lys(Z)² εCH₂); 1.36 (9H, Boc t-Bu); 2.78 (4H, OSu).

### (4e: Synthesis of TFA·H-Gly¹-Lys(Z)²-Gly³-Val⁴-Ala⁵-Pro⁶-OSu (SEQ ID NO: 9))

An amino-terminal of Boc-Gly¹-Lys(Z)²-Gly³-Val⁴-Ala⁵-Pro⁶-OSu (SEQ ID NO: 8) (0.13 g, 0.16 mmol) was deprotected by using TFA to obtain a white powder.
Yield, 0.13 g (90%). ¹H NMR (DMSO-*d₆*, 300MHz): 8.51 (1H, Ala⁵-NH); 8.29 (1H, Lys(Z)² εNH); (1H, Val⁴ NH); (1H, Lys(Z)²NH), 7.20 (1H, Gly³ NH); 7.98 (3H, Gly¹ NH); 7.34 (5H, Z- C₆H₅); 5.00 (2H, Z- -CH₂-); 4.48, 4.33, 4.28, 4.20 (4H, Ala αCH, Pro αCH, Val αCH, Lys(Z) αCH); 3.70 (4H, Gly¹ αCH₂, Gly³ αCH₂); 2.13, 1.93 (2H, Pro⁶ βCH₂); 1.93 (1H, Val⁴ βCH₂); 1.23 (3H, Ala⁵ βCH₃); 1.67 (2H, Lys(Z)² βCH₂); 1.93 (2H, Pro⁶ γCH₂); 0.82 (6H, Val⁴ γCH₃); 1.52 (2H, Lys(Z)² γCH₂); 3.55 (2H, Pro⁶ δCH₂); 1.82 (2H, Lys(Z)² δCH₂); 2.96 (2H, Lys(Z)² εCH₂); 2.78 (4H, OSu).

### (4f: Poly[(Gly¹-Val²-Gly³-Hmb⁴-Ala⁵-Pro⁶)-co-(Gly⁷-Lys(Z)⁸-Gly⁹-Val¹⁰-Ala¹¹-pro¹²)] by polymerization reaction in distilled dioxane)

TFA·H-Gly¹-Val²-Gly3-Hmb⁴-Ala⁵-Pro⁶-OSu (0.430 g, 0.600 mmol) and TFA·H-Gly¹-Lys(Z)²-Gly³-Val⁴-Ala⁵-Pro⁶-OSu (SEQ ID NO: 9) (0.040 g, 0.050 mnol) were mixed in a molar ratio of 0.90 : 0.10, and dissolved in distilled dioxane. Tetraethylamine (90 µl, 0.65 mmol) was added thereto to initiate polymerization. After stirring for 1 week, the molecular weight of the product was confirmed by mass spectrometry. Then, EDC·HCl (0.12 g, 0.65 mmol) and HOBt (0.08 g, 0.65 mmol) were added thereto, followed by stirring for additional 1 week. After that, dialysis was performed for 1 week to remove molecules having molecular weights of 3,500 or less, and the dialysate was freeze-dried to obtain a copolymerized compound. A composition rate of the copolymerization was investigated by ¹H NMR spectrum as shown in Fig. 1. It was confirmed that the composition rate was 0.93:0.07 as apparent from a ratio in integration of a Lys(Z)⁸ signal.
Yield = 35.8 mg; melting point = 164-170 °C; average molecular weight = 6,000 (apparent value obtained by MALDI-TOF mass spectrometry). ¹H NMR (DMSO-*d*₆, 500MHz): 8.42 (Gly³ NH); 8.11 (Gly¹ NH, Ala⁵ NH); 7.60 (Val² NH); 7.33 (Z-, C₆H₅), 4.98 (Z, -CH₂-); 4.75 (Hmb⁴, αCH); 4.50 (Ala⁵ aCH); 4.26 (Pro⁶ aCH); 4.18 (Val², αCH); 3.89 (Gly³ αCH₂); 3.96 (Gly¹ αCH₂); 2.00, 1.81 (Pro⁶ βCH₂); 2.00 (Val² βCH₂); 1.19 (Ala⁵ βCH₃); 2.00 (Pro⁶ γCH₂); 0.82 (Val² γCH₃); 3.59, 3.54 (Pro⁶, δCH₂).

### (4g: Deprotection reaction of Lys(Z)⁸ side chain, and synthesis of poly[(Gly¹-Val²-Gly³-Hmb⁴-Ala⁵-Pro⁶)-co-(Gly⁷-Lys⁸-Gly⁹-Val¹⁰-Ala¹¹-Pro¹²)])

In a 50-ml round-bottom flask, poly[(Gly¹¹-Val²-Gly³-Hmb⁴-Ala⁵-Pro⁶)ₙ-co-(Gly⁷-Lys(Z)⁸-Gly⁹-Val¹⁰-Ala¹¹-Pro¹²)_{1-n]} (10 mg) was dissolved in MeOH and added with palladium carbon powder. An apparatus for catalytic reduction reaction was built, and the atmosphere was replaced with hydrogen gas, followed by stirring. Progression of the reaction was confirmed by an amount of consumption of the hydrogen gas and elimination of the spots of the raw material by TLC, and termination of the reaction was determined. After that, the palladium carbon powder was removed, and a filtrate was concentrated. The residue was added with benzene, and water was removed by azeotropy. The residue was repeatedly subjected to reprecipitation using a solvent system of distilled CHCl₃-petroleum ether. The deprotection of the Lys(Z)⁸ side chain was confirmed by ¹H NMR spectrum.
Yield, 9 mg (90%). ¹H NMR (DMSO-*d*₆, 500MHz): 8.42 (Gly³ NH); 8.11 (Gly¹ NH, Ala⁵ NH); 7.60 (Val² NH); 4.76 (Hmb⁴, αCH); 4.50 (Ala⁵ αCH); 4.27 (Pro⁶, αCH); 4.27 (Val², αCH); 3.90 (Gly³, αCH₂); 3.70 (Gly¹, αCH₂); 2.00, 1.83 (Pro⁶ βCH₂); 2.00 (Val², βCH₂); 2.00 (Hmb⁴ βCH); 1.19 (Ala⁵ βCH₃); 2.00 (Pro⁶ γCH₂); 0.87 (Val² γCH₃); 3.59, 3.50 (Pro⁶ δCH₂).

### Example 5

### (5) Synthesis of poly(Gly¹-Val²-Gly³-Hmb⁴-Pro⁵)

### (5a: Synthesis of Boc-Gly-Hmb-Pro-OBzl)

HCl·H-Pro-OBzl (10.22 g, 42.3 mmol) was put in a 500-ml round-bottom flask and added with distilled chloroform (250 ml), followed by stirring with a magnetic stirrer so that it is dissolved. Then, the reaction mixture was added with NMM (4.70 ml, 42.6 mmol), Boc-Gly-Hmb-OH (13.99 g, 50.8 mmol), and HOBt (7.74 g, 57.3 mmol), and then added with DCC (12.01 g, 58.2 mmol) while being cooling with ice, followed by stirring. After termination of the reaction, DCUrea was removed, and the filtrate was concentrated and added with EtOAc (300 ml), followed by being left standing for 1 hour. DCUrea was removed again, and the obtained filtrate was washed 3 times with an aqueous 10% citric acid solution, once with saline, 3 times with a saturated aqueous NaHCO₃ solution, and once with saline in this order. An organic phase was obtained and added with Na₂SO₄ and dried, followed by concentration. Then, it was purified by silica gel column chromatography using as a developing solvent a mixture of ethyl acetate and benzene (ethyl acetate:benzene = 1:9) to obtain an oil-form product. A few impurities were left, but the product was subjected to the following experiment.
Yield, 7.0 g (68%).

### (5b: Synthesis of Boc-Val-Gly-Hmb-Pro-OBzl)

Condensation reaction was performed by using HCl·H-Gly-Hmb-Pro-OBzl (9.36 g, 22.6 mmol), NMM (2.50 ml, 22.6 mmol), Boc-Val-OH (7.44 g, 34.2 mmol), HOBt (4.66 g, 34.5 mmol), and DCC (7.14 g, 34.6 mmol). Purification was performed by silica gel column chromatography using as a developing solvent a mixture of EtOAc and benzene (EtOAc:benzene = 1:9) to obtain an oil-form product. A few impurities and few target product were left, but the product was subjected to the following experiment.
Yield, 11.8 g (93%).

### (5c: Synthesis of Boc-Gly-Val-Gly-Hmb-Pro-OBzl)

Condensation reaction was performed by using HCl·H-Val-Gly-Hmb-Pro-OBzl (5.03 g, 9.79 mmol), NMM (1.10 ml, 9.97 mmol), Boc-Val-OH (2.82 g, 15.9 mmol), HOBt (2.17 g, 16.1 mmol), and EDC·HCl (3.04 g : 15.7 mmol). Purification was performed by silica gel column chromatography using as a developing solvent a mixture of EtOAc and benzene (EtOAc:benzene = 4:1).
Yield, 2.03 g (34%); melting point = 51-54 °C; [α]_{D}²⁰ = -94.0 deg. (MeOH, c 0.1); electrospray mass spectrometry: 619.5 ([M+H]⁺), 641.4 ([M+Na]⁺).

### (5d: Synthesis of Boc-Gly-Val-Gly-Hmb-Pro-OH)

Boc-Gly-Val-Gly-Hmb-Pro-OBzl (2.51 g, 4.06 mmol) was put in a 300-ml round-bottom flask and dissolved in MeOH (30 ml), and then 1 micro-spatula of palladium carbon was added thereto, followed by stirring. The atmosphere was replaced with hydrogen gas and catalytic reduction was allowed to proceed for about 24 hours. After termination of the reaction, the palladium carbon was removed, MeOH was distilled off, and the residue was added with hexane (30 ml) for crystallization. After that, recrystallization was performed by using EtOAc (20 ml) and hexane (50 ml).
Yield = 1.85 g (86%); melting point = 73-77°C; [α]_{D}²⁰ = -89 deg. (MeOH, c 0.1).

### (5e: Synthesis of Boc-Gly-Val-Gly-Hmb-Pro-OSu)

Boc-Gly-Val-Gly-Hmb-Pro-OH (0.34 g, 0.64 mmol) was put in a 50-ml round-bottom flask and dissolved in 5 ml of DMF. The obtained solution was added with HOSu (0.10 g, 0.87 mmol), and then added with DCC (0.17 g, 0.82 mmol) while being cooled with ice, followed by stirring overnight. After termination of the reaction, generated DCUrea was removed, and the filtrate was concentrated. The residue was added with EtOAc, and DCUrea was again filtrated out. The filtrate was dried with a vacuum pump to obtain a colorless solid.
Yield, 0.36 g (89%); [α]_{D}²⁰ = -79 deg. (MeOH, c 0.1); melting point = 45-49 °C.

### (5f: Synthesis of poly(Gly-Val-Gly-Hmb-Pro) by polymerization reaction in distilled CHCl₃)

TFA·H-Gly-Val-Gly-Val-Pro-ONSu (0.27 g, 0.58 mmol) was put in a 50-ml round-bottom flask and dissolved in distilled CHCl₃ (10 ml). The mixture was added with TEA (0.081 ml, 0.58 mmol) while being cooled with ice, followed by stirring. After 1 hour, the flask was taken out from an ice bath, followed by stirring at room temperature for 2 weeks. After that, dialysis was performed for 1 week with a semipermeable membrane through which molecules having molecular weights of 3,500 or less can pass, and the dialysate was freeze-dried to obtain a colorless powder.
Yield, 53 mg (22%); melting point, 173-186 °C; average molecular weight = 5,000 (apparent value obtained by MALDI-TOF mass spectrometry).

### Example 6

### (6) Synthesis of poly(Gly¹-Thr²-Gly³-Hmb⁴-Pro⁵)

### (6a: Synthesis of Boc-Thr-Gly-Hmb-Pro-OBzl)

Condensation reaction was performed by using HCl·H-Gly-Hmb-Pro-OBzl (11.24 g, 27.1 mmol), NMM (3.30 ml, 29.9 mmol), Boc-Thr-OH (7.40 g, 33.8 mmol), HOBt (3.77 g, 27.4 mmol), and DCC (7.35 g, 35.6 mmol). Purification was performed by silica gel column chromatography using as a developing solvent a mixture of EtOAc and Benzene (EtOAc:Benzene = 4:1) to obtain a colorless oil.
Yield, 6.03 g (38%).

### (6b: Synthesis of Boc-Gly-Thr-Gly-Hmb-Pro-OBzl)

Condensation reaction was performed by using HCl·H-Thr-Gly-Hmb-Pro-OBzl (4.89 g, 9.48 mmol), NMM (1.05 ml, 9.53 mmol), Boc-Val-OH (2.75 g, 15.5 mmol), HOBt (1.28 g, 9.47 mmol), and EDC·HCl (2.75 g, 14.3 mmol). Purification was performed by silica gel column chromatography using as a developing solvent a mixture of EtOAc and Benzene (EtOAc:Benzene = 1:1) to obtain a colorless crystal.
Yield, 4.40 g (75%); [α]_{D}²⁰= -80 deg. (MeOH, c 0.1); melting point = 70-72 °C, electrospray mass spectrometry = 621.4 ([M+H]⁺)₃ 643.4 ([M+Na]⁺).

### (6c: Synthesis of Boc-Gly-Thr-Gly-Hmb-Pro-OH)

Boc-Gly-Thr-Gly-Hmb-Pro-OBzl (1.00 g, 1.61 mmol) was put in a 100-ml round-bottom flask and dissolved in MeOH (15 ml), and then 1 micro-spatula of palladium carbon was added thereto, followed by stirring. Catalytic reduction was allowed to proceed for about 4 hours under hydrogen gas. After termination of the reaction, the palladium carbon was filtrated out, MeOH was removed by concentration, and the residue was added with hexane (30 ml) for crystallization. After that, recrystallization was performed by using EtOAc (10 ml) and hexane (20 ml).
Yield. 0.73 g (86%); [α]_{D}²⁰ = -77 deg. (MeOH, c 0.1); melting point = 96-97°C; electrospray mass spectrometry = 569.3 ([M+Na]⁺).

### (6d: Synthesis of Boc-Gly-Thr-Gly-Hmb-Pro-OSu)

Boc-Gly-Thr-Gly-Hmb-Pro-OH (0.71 g, 1.34 mmol) was put in a 100-ml round-bottom flask and dissolved in DMF (10 ml). The mixture was added with HOSu (0.23 g, 2.01 mmol) and then added with DCC (0.41 g, 2.01 mmol) while being cooled with ice, followed by stirring overnight. After termination of the reaction, DCUrea was filtrated out, and the filtrate was concentrated. The filtrate was added with EtOAc and insoluble DCUrea was removed again. The obtained filtrate was again concentrated to obtain a colorless oil.
Yield: 1.0 g (substantially quantitative).

### (6d: Synthesis of poly(Gly-Thr-Gly-Hmb-Pro) by polymerization reaction in distilled CHCl₃)

TFA·H-Gly-Thr-Gly-Val-Pro-OSu (0.93 g, 1.34 mmol) was put in a 100-ml round-bottom flask and dissolved in distilled CHCl₃ (15 ml). The solution was added with TEA (0.19 ml, 1.36 mmol) while being cooled with ice, followed by stirring. After 1 hour, the flask was taken out from an ice bath, and stirred at room temperature for 2 weeks. After that, dialysis was performed for 1 week with a semipermeable membrane through which molecules having molecular weights of 3,500 or less can pass, and the dialysate was freeze-dried to obtain a colorless powder.
Yield, 31 mg (6%); melting point = 162-176 °C; average molecular weight = 4,000 (apparent value obtained by MALDI-TOF mass spectrometry).

### Example 7

### (7) Synthesis of poly(Gly¹-Ile²-Gly³-Hmb⁴-Pro⁵)

### (7a: Synthesis of Boc-Gly-Hmb-Pro-OBzl)

Boc-Gly-Hmb-OH (10.71 g, 38.9 mmol) was dissolved in distilled CHCl₃ and added with DCC (8.03 g, 38.9 mmol) and HOBt (5.96 g, 38.9 mmol). In another round-bottom flask, a distilled CHCl₃ solution of HCl·H-Pro-OBzl (10.34 g, 35.4 mmol) was prepared, and neutralized with NMM (3.89 ml, 35.4 mmol). Both of the solutions were mixed together and stirred to initiate condensation reaction. The mixture was gradually returned to room temperature and stirred overnight, and concentrated. The obtained residue was added with EtOAc, and precipitated DCUrea was repeatedly removed. The obtained solution was washed sequentially with 10% citric acid, distilled water, saturated NarcO3 distilled water, and saturated NaCl, dried with Na₂SO₄. and concentrated to obtain an oil-form target product. Yield, 15.39 g (94.0%. ¹H NMR (CDCl₃. 300 MHz): 7.33 (5H. -OBzl C₆H₅); 5.13 (2H, -OBzl -CH₂-); 4.84 (1H, Hmb αCH); 4.60 (1H, Gly NH); 4.01 (2H, Gly αCH₂); 3.84 (1H, Pro αCH); 3.62 (2H, Pro δCH₂); 2.20 (2H, Pro βCH₂); 2.04 (1H, Hmb βCH); 2.00 (2H, Pro γCH₂); 1.44 (9H, Boc t-Bu); 1.00 (3H, Hmb γCH₃).

### (7b: Synthesis of Boc-Ile-Gly-Hmb-Pro-OBzl)

Condensation reaction was performed by using Boc-Ile-OH·1/2H₂O (8.86 g, 36.9 mmol), EDC·HCl (6.43 g, 36.9 mmol), HOBt (4.98 g, 36.9 mmol). HCl·H-Gly-Hmb-Pro-OBzl (13.37 g, 33.5 mmol), and NMM (3.69 ml, 33.5 mmol). Purification was performed by column chromatography (EtOAc:benzene = 1:9) to obtain an oil-form target product.
Yield = 17.76 g (92.0%); electrospray mass spectrometry = 576.4 ([M+H]⁺), 598.4 ([M-Na]⁺). NMR (DMSO-*d*₆, 300 MHz): 8.32 (1H, Gly NH); 7.33 (5H, -OBzl C₆H₅); 6.64 (1H, Ile NH); 5.09 (2H, -OBzl -CH₂-); 4.87 (1H, Hmb αCH); 4.38 (1H, Pro αCH); 4.00 (1H, Ile αCH); 3.80 (2H, Gly αCH₂); 3.72 (2H, Pro δCH₂); 2.19, 1.88 (2H, Pro βCH₂); 2.01 (1H, Hmb βCH); 1.88 (2H, Pro γCH₂); 1.66 (1H. Ile βCH); 1.35 (9H, Boc *t*-Bu); 1.14 (2H, Ile γCH₂); 0.92 (3H, Ile γCH₃); 0.89 (3H, Ile δCH₃); 0.78 (3H, Hmb γCH₃).

### (7c: Synthesis of Boc-Gly¹-Ile²-Gly³-Hmb⁴-Pro⁵-OBzl)

Condensation reaction was performed by using Boc-Gly-OH (2.58 g, 14.7 mmol), EDC·HCl (2.83 g, 14.7 mmol), HOBt (1.99 g, 14.7 mmol), HCl·H-Ile-Gly-Hmb-Pro-OBzl (6.88 g, 13.4 mmol), and NMM (1.47 ml, 13.4 mmol). Purification was performed by column chromatography (EtOAc:benzene = 1:10) to obtain an oil-form target product.
Yield, 4.93 g (58.1 %); electrospray mass spectrometry = 633.5 ([M+H]⁺), 655.5 ([M+Na]⁺). ¹H NMR (DMSO-*d*₆*,* 300 MHz): 8.50 (1H, Gly³ NH); 7.64 (1H, Ile² NH); 7.32 (5H, -OBzl C₆H₅); 6.96 (1H, Gly¹ NH); 5.09 (2H, -OBzl-CH₂-); 4.88 (1H, Hmb⁴αCH); 4.37 (1H, Pro⁵ αCH); 4.23 (2H, Pro⁵ δCH₂); 3.98 (2H, Gly³ αCH₂); 3.92 (1H. Ile² αCH); 3.72 (2H, Gly¹ αCH₂); 2.19, 1.80 (2H, Pro⁵ βCH₂); 2.02 (1H, Hmb⁴ βCH); 1.93 (2H, Pro⁵ γCH₂); 1.68 (1H, Ile² βCH); 1.36 (9H, Boc *t*-Bu); 1.14 (2H, Ile² γCH₂); 0.92 (3H, Ile² γCH₃); 0.88 (3H. Ile² δCH₃); 0.79 (3H, Hmb⁴ γCH₃).

### (7d: Synthesis of Boc-Gly¹-Ile²-Gly³-Hmb⁴-Pro⁵-OH)

Boc-Gly¹-Ile²-Gly³-Hmb⁴-Pro⁵-OBzl (4.93 g, 7.79 mmol) was dissolved in MeOH and added with palladium carbon powder. After an apparatus was built, the round-bottom flask was filled with H₂, and then stirred to initiate catalytic reduction reaction. Progression of the reaction was confirmed by elevation of a liquid surface, and the termination of the reaction was determined when spots of the raw materials in TLC were eliminated. After that, the palladium carbon powder was removed, and the filtrate was concentrated, followed by azeotropy with benzene to obtain an oil-form target product.
Yield, 4.34 g (quantitative). ¹H NMR (DMSO-*d*₆, 300 MHz): 8.49 (1H, Gly³ NH); 7.60 (1H, Ile² NH); 6.95 (1H, Gly¹ NH); 4.90 (1H, Hmb⁴ αCH); 4.22 (1H, Pro⁵ αCH); 4.22 (1H, Ile² αCH); 3.98 (2H, Gly³ αCH₂); 3.79 (2H, Pro⁵ δCH₂); 3.53 (2H, Gly¹ αCH₂); 2.07, 1.75 (2H, Pro⁵ βCH₂); 1.97 (1H, Hmb⁴ βCH); 1.97 (2H, Pro⁵ γCH₂); 1.67 (1H, Ile² βCH); 1.36 (9H, Boc *t*-Bu); 1.14 (2H, Ile² γCH₂); 0.98 (3H, Hmb⁴ γCH₃); 0.84 (3H, Ile² γCH₃); 0.79 (3H, Ile² δCH₃).

### (7e: Synthesis of Boc-Gly¹-Ile²-Gly³-Hmb⁴-Pro⁵-OSu)

Boc-Gly¹-Ile²-Gly³-Hmb⁴-Pro⁵-OH (4.34 g, 8.00 mmol) was dissolved in distilled DMF and added with DCC (1.82 g, 8.80 mmol), followed by stirring under cooling with ice. The solution was gradually returned to room temperature and stirred overnight, and then DCUrea was removed. After that, the resultant solution was concentrated and subjected to recrystallization with dehydrated Et₂O/petroleum ether to obtain a target product.
Yield, 4.09 g (79.9%). ¹H NMR (DMSO-*d*₆, 300 MHz): 8.52 (1H, Gly³ NH); 7.62 (1H, Ile² NH); 6.96 (1H, Gly¹ NH); 4.92 (1H, Hmb⁴ αCH); 4.74 (1H, Pro⁵ αCH); 4.23 (1H, Ile² αCH); 3.93 (2H, Gly³ αCH₂); 3.77 (2H, Pro⁵ δCH₂); 3.55 (2H, Gly¹ αCH₂); 2.78 (4H, -OSu); 2.45, 1.99 (2H, Pro⁵ PCH₂); 1.99 (1H, Hmb⁴ βCH); 1.99 (2H, Pro⁵ γCH₂); 1.66 (1H, Ile² βCH); 1.36 (9H, Boc *t*-Bu); 1.04 (2H, Ile² γCH₂); 0.93 (3H, Hmb⁴ γH₃); 0.81 (3H, Ile² γCH₃); 0.76 (3H, Ile² δCH₃).

### (7f: Synthesis of HCl·H-Gly¹-Ile²-Gly³-Hmb⁴ -Pro⁵-OSu)

The amino-terminal of Boc-Gly¹-Ile²-Gly³-Hmb⁴-Pro⁵-OSu (2.47 g. 3.86 mmol) was deprotected by using 4 M HCl/dioxane (9.7 ml) to obtain a target product.
Yield: 2.33 g (quantitative).

### (7g: Synthesis of poly(Gly¹-Ile²-Gly³-Hmb⁴-Pro⁵)

HCl·H-Gly¹-Ile²-Gly³-Hmb⁴-Pro⁵-OSu (1.51 g, 2.62 mmol) was dissolved in distilled DMF, and then tetraethylamine (0.36 ml, 2.62 mmol) was dropped thereto to initiate polymerization. After 2 weeks, the reaction solution was concentrated, dissolved in CHCl₃, and washed sequentially with distilled water and saturated NaHCO₃. After that, crystallization was performed using dehydrated Et₂O, and thus-generated solid was dissolved in distilled water over 3 days. Then, the solution was freeze-dried to obtain a target product.
Yield, 0.67 g (57.8%); melting point, 176-189 °C; number average molecular weight = 2,640 (apparent value obtained by MALDI-TOF mass spectrometry).

### Example 8

### (8) Mass spectrometry by MALDI-TOF

Mass spectrometry by MALDI-TOF was performed to determine the molecular weights of the polymers, that is, the compounds synthesized by the polymerization reaction of the compounds obtained by the present invention. Fig. 2 shows a result for poly(Gly-Val-Gly-Hmb-Ala-Pro). As a result, peaks were observed per about 481 in a high molecular weight region (molecular weights of 3,000 or more) in the spectrum, so it was confirmed that a polymer was produced by the polymerization reaction. From the sample as used in this case, components having molecular weights of 3,500 or less had been removed by dialysis. Signals in the low molecular weight region (molecular weights of 3,000 or less) are ascribed to peaks derived from decomposition in the analysis apparatus or polyvalent ions. In addition, strengths of the peaks in the high molecular weight region vary depending on the strength of applied laser, so the strength does not correspond to molecular weight distribution itself. Actually, components having molecular weights of 10,000 or more were able to be observed by increasing the strength of the laser.

### Example 9

### (9) Circular dichroism spectrum

Temperature-dependent circular dichroism spectra of the compounds obtained by the present invention were measured. From the circular dichroism spectra, information, which is different from that of the visually-observed change in temperature responsibility, can be obtained, for example, a finding regarding a solution structure. Fig. 3 and Fig. 11 show results for poly(Gly-Val-Gly-Hmb-Ala-Pro) and poly(Gly-Ile-Gly-Hmb-Pro), respectively.

In Fig. 3, the spectra showed a change with a circular dichroism point at 212 nm. No aggregation was observed because a diluted aqueous solution (1 mg/ml) was used. As the temperature increased, the negative band at 196 nm decreased. It was considered that this is because the solution structure became more random, as described in the related study of the inventors of the present invention (for example, Oku et al., Journal of Polymer Science, Part A, Polymer Chemistry, 2000, vol. 38, pp. 4524).

In Fig. 11, the spectra showed a change with a circular dichroism point at 220 nm. The spectra showed a negative maximum at 195 nm when the temperature is low, and showed a positive maximum at 210 nm and a negative maximum at 225 nm as the temperature increased. Also, the negative maximum at 195 nm decreased as the temperature increased. This is a tendency similar to the spectra shown in Fig. 3, so it is considered that a similar structural change occurred. No aggregation was observed because a diluted aqueous solution (0.1 mg/ml) was used.

### Example 10

### (10) Observation of temperature responsibility with photograph

The temperature responsibility of the compounds obtained by the present invention was observed by taking photographs of heated aqueous solutions of the compounds. Fig. 4 and Fig. 5 show the results for poly(Gly-Val-Gly-Hmb-Ala-Pro), and Fig. 12 shows the result for poly(Gly-Ile-Gly-Hmb-Pro). Fig. 4 and Fig. 5 showed that an aqueous solution which is transparent at 25 °C became opaque by being heated to 55 °C. Fig. 12 showed that an aqueous solution which is transparent at 0°C became opaque by being heated to 20°C.
These phenomena of being opaque can be repeatedly observed by alternately performing heating and cooling.

### Example 11 I

### (11) Observation of temperature responsibility by apparent absorbance

The temperature responsibility of the compounds obtained by the present invention was observed by measuring apparent absorbance of heated aqueous solutions of the compounds. The observation was performed at a wavelength of 500 nm. The temperature responsibility corresponds to visually-observed light scattering, that is, the phenomena of being opaque. Figures 6 to 9 show the results for poly(Gly-Val-Gly-Hmb-Ala-Pro), poly[(Gly-Val-Gly-Hmb-Ala-Pro)-co-(Gly-Lys(Z)-Gly-Val-Ala-Pro)], poly(Gly-Val-Gly-Hmb-Pro), poly(Gly-Thr-Gly-Hmb-Pro), TFA·H-(Gly-Val-Gly-Hmb-Pro)₅-OH, respectively. The longitudinal axis of the graph represents apparent absorbance instead of transmittance. By the figures, sharpness of temperature responsibility can be compared. With regard to the visual observation, aggregation well proceeded when the apparent absorbance exceeded about 0.1, and it was considered that no further change occurred.

### Example 12

### (12) Observation of reversibility in temperature responsibility

Reversible changes in temperature responsibility of the compounds obtained by the present invention were observed by measuring transmittance of heated aqueous solutions of the compounds. In this case, the observation was performed at a wavelength of 500 nm. The reversible changes correspond to visually-observed light scattering, that is, the phenomena of being opaque. Fig. 13 and Fig. 14 show the results for poly(Gly-Ile-Gly-Hmb-Pro) having different concentrations, respectively. As a result, in a case where the temperature at which the transmittance was 50% was regarded as a transition temperature and the polymer concentration was 10 mg/ml, the transition temperature at heating was 10.2°C and the transition temperature at cooling was 9.2 °C. Under a condition of a higher concentration of 20 mg/ml, the transition temperature at heating was a lower value, that is, 5 °C. Similarly, the transition temperature at cooling was 3.5 °C. In both cases, reversible transition phenomena were observed.

### Example 13

### (13) Visual observation of temperature responsibility

The change in temperature responsibility of the compounds obtained by the present invention was visually observed by heating aqueous solutions of the compounds. Fig. 10 shows the results for the compounds consisting of a certain number (3 or 6) of a -Gly-Val-Gly-Hmb-Ala-Pro- unit, and polymers of poly(Gly-Val-Gly-Hmb-Ala-Pro), poly(Gly-Val-Gly-Hmb-Pro), poly(Gly-Thr-Gly-Hmb-Pro), and poly(Gly-Ile-Gly-Hmb-Pro).

### Industrial Applicability

According to the present invention, there can be obtained a temperature responsive depsipeptide polymer and a temperature responsive composition containing the same. The polymer and composition of the present invention can be used for constituting compositions which are decomposed and absorbed in living bodies, compositions which are decomposed and absorbed in environment such as soil, as well as for cell adhesives, wound-covering materials, microcapsules, biological machines, biosensors, separation membranes, test kits, and the like. In addition, the polymer and temperature responsive composition of the present invention have an advantage of high safety in living bodies.

## Claims

1. A linear depsipeptide polymer comprising a repeating unit selected from the group consisting of:
Gly-Val-Gly-Hmb-Ala-Pro
Gly-Val-Gly-Hmb-Pro
Gly-Thr-Gly-Hmb-Pro, and
Gly-Ile-Gly-Hmb-Pro
said linear depsipeptide polymer comprising 18 or more of amino acid residues and valic acid residues in total,
wherein Hmb represents a valic acid residue represented by the following formula (II) 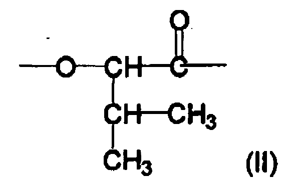 and wherein at least one of the amino acid residues at the N terminal and the C terminal of the linear depsipeptide polymer are deprotected.

2. The linear depsipeptide polymer according to claim 1, which is poly[(Gly-Val-Gly-Hmb-Ala-Pro)-co-(Gly-Lys-Gly-Val-Ala-Pro)].

3. A temperature responsive composition, comprising the linear depsipeptide polymer according to claim 1 or 2.

## Patentansprüche

1. Lineares Depsipeptidpolymer, umfassend eine sich wiederholende Einheit, die ausgewählt ist aus der Gruppe bestehend aus:
Gly-Val-Gly-Hmb-Ala-Pro,
Gly-Val-Gly-Hmb-Pro,
Gly-Thr-Gly-Hmb-Pro und
Gly-Ile-Gly-Hmb-Pro
wobei das lineare Depsipeptidpolymer insgesamt 18 oder mehr Aminosäurereste und Valinsäurereste umfaßt,
wobei Hmb einen Valinsäurerest darstellt, der durch die folgende Formel (II) dargestellt wird 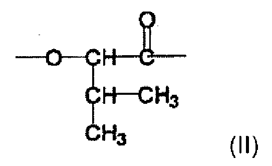 und wobei wenigstens einer der Aminosäurereste an dem N-Terminus und dem C-Terminus des linearen Depsipetidpolymers ungeschützt ist.

2. Lineares Depsipeptidpolymer nach Anspruch 1, das poly[(Gly-Val-Gly-Hmb-Ala-Pro)-co-(Gly-Lys-Gly-Val-Ala-Pro)] ist.

3. Temperaturempfindliche Zusammensetzung, umfassend das lineare Depsipeptidpolymer nach Anspruch 1 oder 2.

## Revendications

1. Polymère depsipeptidique linéaire comprenant une unité répétée choisie dans le groupe consistant en :
Gly-Val-Gly-Hmb-Ala-Pro
Gly-Val-Gly-Hmb-Pro
Gly-Thr-Gly-Hmb-Pro, et
Gly-Ile-Gly-Hmb-Pro
ledit polymère depsipeptidique linéaire comprenant 18 ou plus résidus d'aminoacides et résidus d'acide valique au total,
où Hmb représente un résidu d'acide valique représenté par la formule (II) suivante 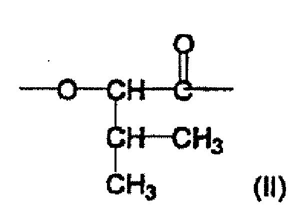 et où au moins l'un des résidus d'aminoacides à l'extrémité N-terminale et à l'extrémité C-terminale du polymère depsipeptidique linéaire est déprotégé.

2. Polymère depsipeptidique linéaire selon la revendication 1, qui est poly[(Gly-Val-Gly-Hmb-Ala-Pro)-co-(Gly-Lys-Gly-Val-Ala-Pro)].

3. Composition sensible à la température comprenant le polymère depsipeptidique linéaire selon la revendication 1 ou 2.
